# EUROPEAN PATENT APPLICATION

(11) **EP 2 610 620 A1**
(43) Date of publication of application: **03.07.2013**
(21) Application number: 11196214.8
(22) Date of filing: 30.12.2011
(51) Int. Cl.: G01N 33/574, A61K 31/00

(54) **Histone deacetylase 10-inhibitor co-treatment in cancer**

(71) Applicant: Deutsches Krebsforschungszentrum, 69120 Heidelberg (DE)
(72) Inventor: The designation of the inventor has not yet been filed
(74) Representative: Dick, Alexander

(57) **Abstract**

The present invention relates to a method of identifying a subj ect being susceptible to a co-treatment with an histone deacetylase 10 (HDAC10)-inhibitor, said subject being in need of and/or being treated with cancer therapy against a cancer, comprising the steps of a) determining the amount of an HDAC10 gene product and/or an autophagy-related gene 4D (ATG4D) product in a cancer sample from said subject, b) comparing the amount of said HDAC10 gene product as determined in step a) with a reference amount for said HDAC10 gene product and/or the amount of ATG4D product as determined in step a) with a reference amount for said ATG4D product, and c) identifying a subject being susceptible to co-treatment with an HDAC10-inhibitor. Moreover, The present invention relates to a method of predicting the risk of recurrence and/or progression of a cancer in an subject treated by cancer therapy, comprising the steps of a) determining the amount of an HDAC10 gene product and/or an ATG4D product in a cancer sample from said subject, b) comparing the amount of said HDAC10 gene product as determined in step a) with a reference amount for said HDAC10 gene product and/or the amount of ATG4D product as determined in step a) with a reference amount for said ATG4D product, and c) predicting the risk of recurrence and/or progression of a cancer treated by cancer therapy. The present invention also relates to a device for identifying a subject being susceptible to co-treatment with an HDAC10-inhibitor, a kit adapted to carry out the method of the present invention. Finally, the invention relates to 2-(4-butoxyphenyl)-N-hydroxyacetamide (bufexamac) for use in treating cancer in a subject, wherein said bufexamac is to be applied in a co-treatment with cancer therapy.

SEQUENCE LISTING

<110>  Deutsches Krebsforschungszentrum

<120>  Histone Deacetylase 10-inhibitor Co-Treatment in Cancer

<130>  DK10777EP

<160>  21

<170>  PatentIn version 3.5

<210>  1
<211>  6223
<212>  DNA
<213>  Homo sapiens

<400>  1
gcgcagctcc gagggctggc ggctcggggc gccgaggctt ccgcgcccgc tggttcctcg      60

ctccggggcg gagctcgcga tagcgaccgg gagcagggcg cggggcggga cccaggtccg     120

aggcgaggaa gccggaagcc aggcgcgggg agcctccccc ttcgactgca gcctcgctcc     180

gtgccttctg cgcgcctggg atcccggagc ctgcctaggt tctgtgcgct cccgcccagg     240

ccggtgcccg ccgcccgcct gcgccccagg caggtcccag gcctccggct gctcccggcc     300

gaaggtgggg acaggcagtg gcaggcacca ctagcgaggg cgtttgggaa cccagggtga      360

ccacggcgca gccatgggga ccgcgcttgt gtaccatgag gacatgacgg ccacccggct     420

gctctgggac gagtaagtgg gacctggccg aggtggggtg cggggaggga cctctgaact     480

gagctcctca tctgccccag ccccgagtgc gagatcgagc gtcctgagcg cctgaccgca     540

gccctggatc gcctgcggca gcgcggcctg gaacagaggt gtctgcggtt gtcagcccgc     600

gaggcctcgg aagaggagct gggcctggtg cacaggtcag gacgggggca ggccccacga     660

tggggggcgg ggcgggcagc ccagctagaa acctaagaac ctgggcctac cccgagcctg     720

ccagcctcga atacagagcc tctccctatg cacctcgggt gggctcacag gcttgaggaa     780

gtgacagggt ggccactccc accatgaatc ccaggtcccc gcctcccgcc cccctgctcc     840

tgctggtgac cctgcctctc ctgcctgccc atctgcatgc agcccagagt atgtatccct     900

ggtcagggag acccaggtcc taggcaagga ggagctgcag gcgctgtccg gacagttcga     960

cgccatctac ttccacccgg tgcgtgtccc cggacacact cacctgtgca gacacacatg    1020

cacgtgcctt tgagcgttca cagccacggc cgccaacccc agccagtgtc ccctggtcca    1080

tacacatgac attgccctgc acacctgcct gctggcactg ccagagcctg gggctggcct     1140

gttctgctca gcagctggtg tcttgcagcc tggcacaagt gtgggtatgg cctgcaaagg    1200

gcagcaggga aggcaggggg ctcatctgcg cccctgcgcc cacagagtac ctttcactgc    1260

gcgcggctgg ccgcaggggc tggactgcag ctggtggacg ctgtgctcac tggagctgtg    1320

caaaatgggc ttgccctggt gaggtgagag ctgccccttc ccctggctgc tcctccgtgg    1380

gtggccctgt gaggggccag ggcgaccctg acgtgaccca aatcctactc tcacgccgta    1440

ggcctcccgg gcaccatggc cagagggcgg ctgccaacgg gttctgtgtg ttcaacaacg 1500

tggccatagc agctgcacat gccaagcaga aacacgggct acacaggtgt gtgccaggct 1560

ggctgcaggg gggtgggccc aggccacaca gcaaggggca gcgcaggagg cgagagaacc 1620

cagccagcac cttcccatcc ctggccgggc tcctggaggt gggaggccct gcccctttgc 1680

caccagcgct gtctgtaccc aggatcctcg tcgtggactg ggatgtgcac catggccagg 1740

ggatccagta tctctttgag gatgacccca ggtgagcagg ggtgcggaaa ccacccttcc 1800

ctgcagcccc agtttttggg tgcacagcct ggcctgggaa aatccagggg tgagatacag 1860

acatgtgctc aggggtcagc gttctacagg gagctgtaga gctgttgagg gtcctgacct 1920

ttgacctctg gccctggctg gctgttggca gcgtccttta cttctcctgg caccgctatg 1980

agcatgggcg cttctggcct ttcctgcgag agtcagatgc agacgcagtg gggcggggac 2040

agggcctcgg cttcactgtc aacctgccct ggaaccaggt gcccaccctc aagccccagg 2100

tccccaccca gccccctccc cctgggcccc tcccctcccc cacagttcac cctcaagccc 2160

caggtcccca cccaccccct cccccgggcc cctcccctgc cccacagttc aggatgtagc 2220

ctgggatgtc ctgccaggtt gggatgggaa acgctgacta cgtggctgcc ttcctgcacc 2280

tgctgctccc actggccttt gaggtgactg cacggagggc tcccccaggg cagccgggtg 2340

ggagggggac cttgtgcctg gagtgcgcca gtgaaggcag gaggagggtt atgagctgtc 2400

ccactcctgt gtggctgggg aaggtgctca ggaggggatg ggatgggcct ggctcgccct 2460

cacctgggct ccagcccctc tctgtcgacc ctccccactc cggcctggcc tgtagtttga 2520

ccctgagctg gtgctggtct cggcaggatt tgactcagcc atcggggacc ctgaggtgag 2580

ggccttgcct tgccagggag ggcttctggg gtcctggggc ccaggtcccc catcacacat 2640

ggctctggtt ccaggggcaa atgcaggcca cgccagagtg cttcgcccac ctcacacagc 2700

tgctgcaggt gctggccggc ggccgggtct gtgccgtgct ggaggtgact ggggaggcca 2760

cacagggagg gtctctgggc cagaggcctg ggggtcagga gcatgggagc caggtgccca 2820

cggggtattt gggaggggcg tcctgcccgg gatagagcca ggcaggggct ctcccggtga 2880

catcccatcc cagtgatatc tctgccccaa tcatgtctcc accctcctcc cagggcggct 2940

accacctgga gtcactggcg gagtcagtgt gcatgacagt acagacgctg ctgggtgacc 3000

cggccccacc cctgtcaggg ccaatggcgc catgtcagag gtgcgagggg aggtagggcg 3060

ggcagggccg gggtgtgaga ccaggtgtca cgactgttgt gtctccctca cagtgcccta 3120

gagtccatcc agagtgcccg tgctgcccag gccccgcact ggaagagcct ccagcagcaa 3180

ggtcagtgtg gccgggtgct aggctgcagg atcccacctc gggctcctgt gcagtggcta 3240

tgactccgaa ctgtccctag atgtgaccgc tgtgccgatg agccccagca gccactcccc 3300

agaggggagg cctccacctc tgctgcctgg gggtccagtg tgtaaggcag ctgcatctgc 3360

accgagctcc ctcctggacc agccgtgcct ctgccccgca ccctctgtcc gcaccgctgt 3420

tgccctgaca acgccggata tcacattggt tctgccccct gacgtcatcc aacaggaagc 3480

gtcagccctg agggaggaga cagaagcctg ggccaggtgg gcagggctgg cctggggaga 3540

aggatggggc cgcatctaca cctgtgcctg tgtgtgcctg acctgcggtc actccgtgtc 3600

tgtgtgtccc tctggtctgt ccaccacagg ccacacgagt ccctggcccg ggaggaggcc 3660

ctcactgcac ttgggaagct cctgtacctc ttagatggga tgctggatgg gcaggtggga 3720

gcacctggga ggggttgggg atgctgtgga cctaggggca gggaccagga cctgccaacg 3780

agatgtggcc tgtccagagg agggctgggt ggtgcagaac cacatggatg tgaacattgc 3840

cttgtgccag gaactgttcc cagaacagcc ccaggggtgg ggatcactag tgtgtctgac 3900

ctctgacctc agcttcttca gttaaatgct ctttggcaga gaggtgaagg gggtgattta 3960

aagtatacat ttaggcaggg tgcagtggct catgcctgta ataacggcag tttggggggc 4020

caaggtggga gaaacgcttg agcccaggag ttcaagacca gcctagagaa catatggaga 4080

ccctgtcttc acaaaaaata aaaaagtcag ccaagcgtgg tggtgtgtgc ctgtagttac 4140

ttaggaggct gaggcaggac gatcgcttaa gcccaggagt ttgaggctgt ggtgagctac 4200

aattgcgcca ctgcacgcca gcctggctac agaacgagac cctgcctctc taaaaaaaaa 4260

aaaaaaaatt atagacgtaa gcatcttatt tacagaaatg gagtgattga ccttagtgta 4320

agaagatgtg tggctgacag tagccgcaga gacacaccga atgaagggaa ctgtggatct 4380

gtttacctgg cttgggagtt ggttggtgcc tcattctcca ctgtttattc atcccacagg 4440

tgaacagtgg tatagcagcc actccagcct ctgctgcagc agccaccctg gatgtggctg 4500

ttcggagagg cctgtcccac ggagcccaga ggtaagcctc acttggccat cctgagcctc 4560

tgacggcagg acctgtgggc acccctatcc tggctggcca agcacagaga gttctgggaa 4620

tggactctgg acaacatgaa cacctgtcct gaggccagct cttcttccag gctgctgtgc 4680

gtggccctgg gacagctgga ccggcctcca gacctcgccc atgacgggta ggactctcag 4740

gtcttgcata tgtccagcca tgcatgtgga cgggcatgtg atggacgtgt gcctgggccc 4800

cagggtgacg ggtgggtggg gcactgtgtg gtactgaatg gagccttcct cagctggggt 4860

gtgggagaag ggctgctgga ggcaatgcca ggtgggtctc cagcacagag gctcagcagt 4920

cacagcaccc ctgcccatgg ccccgtgaat gctcttccac ctctgcctct gcggtttggg 4980

ctcaggaggg tgagcagggc cttacactta tgcacctgtg tcctggaagg aggagtctgt 5040

ggctgaacat caggggcaag gaggcggctg ccctatccat gttccatgtc tccacgccac 5100

tgccagtggt gagtgaccat gccacgcggc aaggcaggtg ctggggagcc catggatgtg 5160

tacagctgct gtttgctggc attgtcatga tagcaggcct ctggtccttc ctgtctccct 5220

gctcctactc caggggccct gagattggga ggttgggagg ccagagccag caggtgtccg 5280

tagatgagga gctgggctct gctgtcccca cagatgaccg gtggtttcct gagctgcatc 5340

ttgggcttgg tgctgcccct ggcctatggc ttccagcctg acctggtgct ggtggcgctg 5400

gggcctggcc atggcctgca gggcccccac gctgcactcc tggctgcaat gcttcggggg 5460

ctggcagggg gccgagtcct ggccctcctg gaggaggtaa gctgggcagg gtggaggtgc 5520

tgcggggtgg gacgagggaa aggaccagtg actgcttccg tcttcgcccc tggtccagaa 5580

ctccacaccc cagctagcag ggatcctggc ccgggtgctg aatggagagg cacctcctag 5640

cctaggccct tcctctgtgg cctccccaga ggacgtccag gccctgatgt acctgagagg 5700

gcagctggag cctcagtgga agatgttgca ggtgaggctg actgcggagg catggccctg 5760

ccctggggcc tggctctgcc gcacagatcg gtggggaggg cagggcaggg gagtgccctg 5820

aaccagctcc agcgccggag gccaactgcg cgcctcttct cggctcctcc tgtagtgcca 5880

tcctcacctg gtggcttgaa atcggccaag gtgggagcat ttacaccgca gaaatgacac 5940

cgcacgccag cgccccgcgg ccgcgatccg gaccccaagc ccacggctcc ctcgactctg 6000

gggcacggaa ccccgcccac tcccaatccc cgcgccccgc cctctcccac ccgtgcttcc 6060

cccgctccac ccctcacctc acctcgcccc cgccccaccc atcgcgcccc ggcccgtccc 6120

atcgaggccc atgcaaccca cgctcggtcc cgttccggcc cctgcgcccc gctgcgcttc 6180

gctccccgcc cttgcgccgt tagtaaacat cgctcaaacg aaa 6223

<210> 2
<211> 2388
<212> DNA
<213> Homo sapiens

<400> 2
gcggggagcc tcccccttcg actgcagcct cgctccgtgc cttctgcgcg cctgggatcc 60

cggagcctgc ctaggttctg tgcgctcccg cccaggccgg tgcccgccgc ccgcctgcgc 120

cccaggcagg tcccaggcct ccggctgctc ccggccgaag gtggggacag gcagtggcag 180

gcaccactag cgagggcgtt tgggaaccca gggtgaccac ggcgcagcca tggggaccgc 240

gcttgtgtac catgaggaca tgacggccac ccggctgctc tgggacgacc ccgagtgcga 300

gatcgagcgt cctgagcgcc tgaccgcagc cctggatcgc ctgcggcagc gcggcctgga 360

acagaggtgt ctgcggttgt cagcccgcga ggcctcggaa gaggagctgg gcctggtgca 420

cagagtacct ttcactgcgc gcggctggcc gcaggggctg gactgcagct ggtggacgct 480

gtgctcactg gagctgtgca aaatgggctt gccctggtga ggcctcccgg gcaccatggc 540

cagagggcgg ctgccaacgg gttctgtgtg ttcaacaacg tggccatagc agctgcacat 600

gccaagcaga aacacgggct acacagcgct gtctgtaccc aggatcctcg tcgtggactg 660

ggatgtgcac catggccagg ggatccagta tctctttgag gatgacccca gcgtccttta 720

cttctcctgg caccgctatg agcatgggcg cttctggcct ttcctgcgag agtcagatgc 780

agacgcagtg gggcggggac agggcctcgg cttcactgtc aacatgccct ggaaccaggt 840

tgggatggga aacgctgact acgtggctgc cttcctgcac ctgctgctcc cactggcctt 900

tgagtttgac cctgagctgg tgctggtctc ggcaggattt gactcagcca tcggggaccc 960

tgaggggcaa atgcaggcca cgccagagtg cttcgcccac ctcacacagc tgctgcaggt 1020

gctggccggc ggccgggtct gtgccgtgct ggagtgccct agagtccatc cagagtgccc 1080

gtgctgccca ggccccgcac tggaagagcc tccagcagca agatgtgacc gctgtgccga 1140

tgagccccag cagccactcc ccagagggga ggcctccacc tctgctgcct gggggtccag 1200

tgtgtaaggc agctgcatct gcaccgagct ccctcctgga ccagccgtgc ctctgccccg 1260

caccctctgt ccgcaccgct gttgccctga caacgccgga tatcacattg gttctgcccc 1320

ctgacgtcat ccaacaggaa gcgtcagccc tgaggaggga gacagaagcc tgggccaggc 1380

cacacgagtc cctggcccgg gaggaggccc tcactgcact tgggaagctc ctgtacctct 1440

tagatgggat gctggatggg caggtgaaca gtggtatagc agccactcca gcctctgctg 1500

cagcagccac cctggatgtg gctgttcgga gaggcctgtc ccacggagcc cagaggctgc 1560

tgtgcgtggc cctgggacag ctggaccggc ctccagacct cgcccatgac gggaggagtc 1620

tgtggctgaa catcaggggc aaggaggcgg ctgccctatc catgttccat gtctccacgc 1680

cactgccagt gatgaccggt ggtttcctga gctgcatctt gggcttggtg ctgcccctgg 1740

cctatggctt ccagcctgac ctggtgctgg tggcgctggg gcctggccat ggcctgcagg 1800

gcccccacgc tgcactcctg gctgcaatgc ttcgggggct ggcagggggc cgagtcctgg 1860

ccctcctgga ggagaactcc acaccccagc tagcagggat cctggcccgg gtgctgaatg 1920

gagaggcacc tcctagccta ggcccttcct ctgtggcctc cccagaggac gtccaggccc 1980

tgatgtacct gagagggcag ctggagcctc agtggaagat gttgcagtgc catcctcacc 2040

tggtggcttg aaatcggcca aggtgggagc atttacaccg cagaaatgac accgcacgcc 2100

agcgccccgc ggccgcgatc cggaccccaa gcccacggct ccctcgactc tggggcacgg 2160

aaccccgccc actcccaatc cccgcgcccc gccctctccc acccgtgctt cccccgctcc 2220

acccctcacc tcacctcgcc cccgccccac ccatcgcgcc ccggcccgtc ccatcgaggc 2280

ccatgcaacc cacgctcggt cccgttccgg cccctgcgcc ccgctgcgct tcgctccccg 2340

cccttgcgcc gttagtaaac atcgctcaaa cgaaaaaaaa aaaaaaaa 2388

<210> 3
<211> 669
<212> PRT
<213> Homo sapiens

<400> 3

Met Gly Thr Ala Leu Val Tyr His Glu Asp Met Thr Ala Thr Arg Leu
1               5                   10                  15

Leu Trp Asp Asp Pro Glu Cys Glu Ile Glu Arg Pro Glu Arg Leu Thr
            20                  25                  30

Ala Ala Leu Asp Arg Leu Arg Gln Arg Gly Leu Glu Gln Arg Cys Leu
        35                  40                  45

Arg Leu Ser Ala Arg Glu Ala Ser Glu Glu Glu Leu Gly Leu Val His
    50                  55                  60

Ser Pro Glu Tyr Val Ser Leu Val Arg Glu Thr Gln Val Leu Gly Lys
65                  70                  75                  80

Glu Glu Leu Gln Ala Leu Ser Gly Gln Phe Asp Ala Ile Tyr Phe His
                85                  90                  95

Pro Ser Thr Phe His Cys Ala Arg Leu Ala Ala Gly Ala Gly Leu Gln
            100                 105                 110

Leu Val Asp Ala Val Leu Thr Gly Ala Val Gln Asn Gly Leu Ala Leu
        115                 120                 125

Val Arg Pro Pro Gly His His Gly Gln Arg Ala Ala Ala Asn Gly Phe
    130                 135                 140

Cys Val Phe Asn Asn Val Ala Ile Ala Ala Ala His Ala Lys Gln Lys
145                 150                 155                 160

His Gly Leu His Arg Ile Leu Val Val Asp Trp Asp Val His His Gly
                165                 170                 175

Gln Gly Ile Gln Tyr Leu Phe Glu Asp Asp Pro Ser Val Leu Tyr Phe
            180                 185                 190

Ser Trp His Arg Tyr Glu His Gly Arg Phe Trp Pro Phe Leu Arg Glu
        195                 200                 205

Ser Asp Ala Asp Ala Val Gly Arg Gly Gln Gly Leu Gly Phe Thr Val
    210                 215                 220

Asn Leu Pro Trp Asn Gln Val Gly Met Gly Asn Ala Asp Tyr Val Ala
225                 230                 235                 240

Ala Phe Leu His Leu Leu Leu Pro Leu Ala Phe Glu Phe Asp Pro Glu
                245                 250                 255

Leu Val Leu Val Ser Ala Gly Phe Asp Ser Ala Ile Gly Asp Pro Glu
            260                 265                 270

Gly Gln Met Gln Ala Thr Pro Glu Cys Phe Ala His Leu Thr Gln Leu

        275                 280                 285

Leu Gln Val Leu Ala Gly Gly Arg Val Cys Ala Val Leu Glu Gly Gly
    290                 295                 300

Tyr His Leu Glu Ser Leu Ala Glu Ser Val Cys Met Thr Val Gln Thr
305                 310                 315                 320

Leu Leu Gly Asp Pro Ala Pro Pro Leu Ser Gly Pro Met Ala Pro Cys
                325                 330                 335

Gln Ser Ala Leu Glu Ser Ile Gln Ser Ala Arg Ala Ala Gln Ala Pro
            340                 345                 350

His Trp Lys Ser Leu Gln Gln Gln Asp Val Thr Ala Val Pro Met Ser
        355                 360                 365

Pro Ser Ser His Ser Pro Glu Gly Arg Pro Pro Pro Leu Leu Pro Gly
    370                 375                 380

Gly Pro Val Cys Lys Ala Ala Ala Ser Ala Pro Ser Ser Leu Leu Asp
385                 390                 395                 400

Gln Pro Cys Leu Cys Pro Ala Pro Ser Val Arg Thr Ala Val Ala Leu
                405                 410                 415

Thr Thr Pro Asp Ile Thr Leu Val Leu Pro Pro Asp Val Ile Gln Gln
            420                 425                 430

Glu Ala Ser Ala Leu Arg Glu Glu Thr Glu Ala Trp Ala Arg Pro His
        435                 440                 445

Glu Ser Leu Ala Arg Glu Glu Ala Leu Thr Ala Leu Gly Lys Leu Leu
    450                 455                 460

Tyr Leu Leu Asp Gly Met Leu Asp Gly Gln Val Asn Ser Gly Ile Ala
465                 470                 475                 480

Ala Thr Pro Ala Ser Ala Ala Ala Ala Thr Leu Asp Val Ala Val Arg
                485                 490                 495

Arg Gly Leu Ser His Gly Ala Gln Arg Leu Leu Cys Val Ala Leu Gly
            500                 505                 510

Gln Leu Asp Arg Pro Pro Asp Leu Ala His Asp Gly Arg Ser Leu Trp
        515                 520                 525

Leu Asn Ile Arg Gly Lys Glu Ala Ala Ala Leu Ser Met Phe His Val
    530                 535                 540

Ser Thr Pro Leu Pro Val Met Thr Gly Gly Phe Leu Ser Cys Ile Leu
545                 550                 555                 560

Gly Leu Val Leu Pro Leu Ala Tyr Gly Phe Gln Pro Asp Leu Val Leu
                565                 570                 575

Val Ala Leu Gly Pro Gly His Gly Leu Gln Gly Pro His Ala Ala Leu
            580                 585                 590

Leu Ala Ala Met Leu Arg Gly Leu Ala Gly Gly Arg Val Leu Ala Leu
        595                 600                 605

Leu Glu Glu Asn Ser Thr Pro Gln Leu Ala Gly Ile Leu Ala Arg Val
    610                 615                 620

Leu Asn Gly Glu Ala Pro Pro Ser Leu Gly Pro Ser Ser Val Ala Ser
625                 630                 635                 640

Pro Glu Asp Val Gln Ala Leu Met Tyr Leu Arg Gly Gln Leu Glu Pro
                645                 650                 655

Gln Trp Lys Met Leu Gln Cys His Pro His Leu Val Ala
            660                 665

<210>  4
<211>  9448
<212>  DNA
<213>  Homo sapiens

<400>  4
ctggggacgg gggccgagta gcgccttccc cgggccccgt gaaccggctg cgggtcgccc      60

ttggggggca gcggccgcag ccccccacct gggccctcgg tccgccctcc cggcgcgtcc      120

atgaactcag tgtcgccggc cgccgcgcag taccggagca gcagcccgga ggacgcgcgc      180

cgccggcccg aggcccgcag gccgcggggt cccagaggcc cagaccccaa cggcctgggg      240

ccttccggag ccagcggccc cgctcttggc tctcccgggg ctggcccgag tgagccggac      300

gaagtggaca agttcaaggc caagttcctg acagcctgga acaacgtcaa gtacggtgag      360

gagggggccc ggagatcgtg ggggtgtcgg gggccgttga ggaaaaccag accccggtcc      420

tgccagttat cccacccttg ttcccgtcct gagtcacctg tgggtcccct ccctgcccgg      480

cccagggtgg acctgtggca ggtccaatag tgttttcaag ccccgcccat tcccctgtgt      540

gaccttgggc aagatccttt gcttcctggc gcctccgttt ttgcccctct ctgtaaaatg      600

ggtacaataa tcagaactac cttccttcat cctgaattga tggtggacac tcctgtagct      660

gtcactgggg gccccacctc cgacggaatc cccggagcca ccccggagat aggaactagg      720

atcgtgccca tttcacagat gggggaatgg aagcgctgtt gctgtcatgc aagtgcttca      780

tctcgctggg cgctgcccct acgtctcccc aggttgggtg gttaaaagcc ggaccagctt 840
tagcaagatc tccagcatcc acctctgtgg ccgccgctac cgtttcgagg gcgagggtga 900
gctggtggtt gggaccaggg ctgggggagg cctctccacg cccgccggag tgtccctggt 960
ggcagctgac agacccgctc ttgtaggtga catacagcgt ttccagcggg actttgtgtc 1020
ccgcctgtgg ctcacatacc gccgggactt cccgcccctt cctgggggct gcctgacctc 1080
ggactgtggc tgggggtgca tgttacgcag cggccagatg atgctggcac agggccttct 1140
gctgcatttc ctgcccagag gtgagccata gggggaaggg ggtgcactag gagtacaggg 1200
tcaactgcca tatcagcagt attagaatta cttaccaact aagagttcaa agtcacgtta 1260
gaattgtgtg tcaagtaagt tttggagaga tgagagttgt ttataagagt ttcagccggg 1320
gttggaaacg gtggctcatg cctgtaatcc tagcactttg ggaggctgag gcggatggat 1380
cccttgcggt caggagttca agaccagcct ggccaatatg atgaaacccc atctctacta 1440
aaaatacaaa aattagccgg ttgtggtggc acacgcctgt aatcccagct actcccgagg 1500
ctgaggtggg agaatctctt gaacccagga ggcggaggtt acagcaagcc aagatcgcac 1560
cactgcactc cagcctgggc aacagaggga gactctgtgt caaaaataaa aataaaaaaa 1620
agtttcacca ggcacggtgg ctcatgcctg taatcccagc actttgggag gctgaggctg 1680
gtggatcatg aggtcaggag ttcaagacca gcctggccaa gatggtgaaa ccccatctct 1740
actaaaaata caaaaattag ctgggtgtgg tggcaggcac ctgtaatttg gcaaggtaaa 1800
gcagagaatt gcttgaaccg ggaggcggga ggttgcagtg agccgagatc cgtgccactg 1860
cactccagcc tgggtggcag agcgagactc agtctcaaaa aaaaaaaaag agttttggct 1920
gggcactgtg ctgcatgctt gtaatcccag ctacctggga ggctgaggtg ggaagattgc 1980
tcgtgcccag gtgttcaagt ctgcagtgaa ctatgatcct gccactgcac tccaacccga 2040
gtgacagaat gagactccaa caataacagg tcgagtgtgg tggctcatgc ctgtaatccc 2100
agcacattag gaggctgagg caggaggatc gcttgagcat aggcattcga caccagccta 2160
ggccacatga caaggctcct gtctctacaa aaaaatttta aaaattagct gagtgtggtg 2220
gcatgtgcct gtgatcccaa ctactcagga ggctgaggcg ggagaatctg ttgagctcag 2280
gatttagagg ctgtagtgag acgtgattgt gccactgcac tccagtctgg atgacagtga 2340
gatcctgtct ttatttattt attttattta ttattattat tatttttttt tttgagactg 2400
agtcttcctc tatcatcctg gctgaagtgc agtggagaga tcttggctca ctgcaacctc 2460
tgcctcccag gttcaagcga ttctcctgcc tcagcctcct gagtagctgg gattacaggt 2520
gcccgccacc acgcttgtct actttttgta tatttttttt tttatcagag aaggggtttc 2580
accatgttgg ccaggctggt ctcgaactcc aggcctcaag tgatccacct gccttggtct 2640
cccaaagtgc tgagattaca ggcatgagcc accgcgtctg gctgagatcc tgtctttaag 2700

taacaaaaaa taaataaata aataaggata agtatatgtt tcaggaatat gtaaattctg 2760

ttgaattacg gggttcagga gcattcgcat tgtgtgcggt gcatgtgtag atgggacctc 2820

tgccccccac acactagcac tgtttgacta tgtgctccat tccaccagac tggacatggg 2880

ccgagggcat gggcctgggc ccccctgagc tgtcagggtc agcctctccc agccggtacc 2940

atgggcctgc ccgctggatg cccccacgct gggcccaggg tgcccctgag ctggagcagg 3000

aacgccggca ccggcagatt gtgtcctggt tcgccgacca cccccgggcc ccctttggcc 3060

tacaccggct ggtggagctt gggcagagct caggcaagaa ggcaggtgac tggtatgggc 3120

catcgctagt ggcacacatc ctcaggtgag ggctgctgca gggatcacgg gagttgctgg 3180

gtacccgcag gcactcaggc cttccctcct gcccccagga aagccgtgga gagctgctcc 3240

gacgtcaccc gcctggtggt gtacgtttct caggactgca caggtaaggg gctgggagcc 3300

aagatcacag gggccccacc ctgagccaga ctctgcctta cccgattctt agagtgcatc 3360

ttcctcagag gctggaacaa gttgtggggc agagggacac tggggaagga tgcaaggcct 3420

gtgcagtggg agatagtccc tcttctccgt ctctccctct ggaacaagaa acttcagggg 3480

cttctccttg agtgatagaa tagagacata aggccaggtg tggtggcaca tactgtagtc 3540

tcagctactt gggaggctga ggtgggagga ttgcttgaac ccaggagttc gaggttacag 3600

tgagctgtca ttatgccatt gcactccagc ctgggcgaca gagtaggatc ctgtcgaaaa 3660

aaaaaaaaaa aaaaaaaaga ggccaggcgc agtggctcac gcctgtaatc ccagcacttt 3720

gggaggccaa ggtgggtgga tcacctgagg tcaggtcact ctgtcaccca ggcttcagtg 3780

cagaggcacg atctcggctc actgcaacct ctgcctcctg ggttcaagcg attctcctgc 3840

ctcagcctct caaatagctg ggattacagg cacgcaccac cataccaggc taatttttct 3900

atttttagta gagatggggt ttcaccatgt tgacaaggcc ggtcttgaac tcctgacctc 3960

aggtgattcg cccactttgg cctcccaaag tgctgggatt acaagcttga gccactgtgc 4020

ccagcccctg taattttttt tttttttttt tttttttttt tttttttttt ttttttttga 4080

gacagagtct cactctgtca cccaggctgg agtgcagtgg cacagtcttg gctcactgca 4140

acctccgcct cctgagttcg agtgattctc ctgcctcagc ctcccaatag ctgggactac 4200

aggcgcgtgc catcacgccc agctaatttt ttttgtattt ttagtagagt tggggtttca 4260

ctgtctttgc cagtatggtc tctcgatctc ctgacctcat gatcccccct cctcggcctc 4320

ccaaagtgct gggattacaa gctggagcca ccgtgcccgg ctacctctgt aaatttttta 4380

tgcaaaattg taggtgtttg ggaaaagatt cgattttcag agaaccttat ctccaaaatg 4440

ggtctaatct acttctctag gagcacaaaa tgggcttacg tagctgcaag gcagggggca 4500

ggagaatggg cccaagtaaa tgggactgga ggaggaggga cataccagga tatttggttt 4560

aggggctgag tgcccagtgc tccagcttct gtattggcaa accgaggcat ggagtcactg 4620

aggacctgtc aggcatgggg ctggggagga gcttgagaga acagtctcta cctctccctt 4680

ctgtatatac ttttggtcta catcccaggg cctcagttgc ccactctgta aaatggggat 4740

gtccacacct gcctggaggt cctgggattt tctgggggtg tttggggggc actagaatga 4800

tgattttggt tagtgatgac agtgttgccc tctgagaaat tgcacggttg aggttgagcc 4860

cctggggtta gagggctacg ctggggcttg aagggtggca agaaggtgac ctgctgctgt 4920

cccgtccctc cagtgtacaa ggcggatgtg gcacgcctgg tggccaggcc agaccccaca 4980

gccgagtgga agtctgtggt catcctggtg cccgtgcgac tgggtggcga gactctcaac 5040

cccgtgtatg tgccctgcgt gaaggtaggt tcagctgaat tctaggacac ctccacctgg 5100

gagccctcca gacttgttta gtttgtgtta gggctgctgt ttgtgcagcc ctcatcactt 5160

tttttttttt gagatggagt ttcgctcttg ttgcccaggc tggaggtcaa taacgtgatc 5220

tcggctcgct gcaacctccg cctcctgggt tcaagcgatt ttcttgcctc agcctcccaa 5280

gtagttagga ttacaggcat gcgccaccac gaccacctaa ttttgtactt ttagtagaga 5340

cagggtttca ccattttggt caggctgatc ttgaactcct aacctcaggt catctgccca 5400

cctcggcctc ccaaagtgct gggataacag gcgtgagcca ccacacgggc catccctttt 5460

tttttgagac agagtcttgc tcttttgccc aggctggagt gcagtggcac agtcttggct 5520

cattgcaacc tccgcctccc gagttcaagc aattctcctg cctcagtctc ccaggtagct 5580

gggactacag gcgtgtgcca ccacgcccgg ctaacttttg tatttttagt agagacgggg 5640

tttcgccatg ttggccaggc tggttctcaa actcctgacc tcaggtgatc cacccacctc 5700

agcctcccaa agtgctggga ttacaggtgt gagccaccac gcccggccca tcccttcttt 5760

ttttatacta tgcagcacct gctatgtgtg ctccccactg tcccgactgc tggggattca 5820

gaggggaaca gaataggcag gagttctgag tgtgcctgct gggagcagac gttctgatgg 5880

gaggatgctc agcagtggct atgtaaccaa agtagcatca ctgtttggaa tgaagccagg 5940

cacagtggct caggcctata atcccagcac tttgggaggc cgaggcagga ggatcatttg 6000

agaccagtct gggcaacata gtgagacccc catctctaca caattttttt tgaaaatatt 6060

cgagtctcac tctcttgccc acgctggagt gcagtgacac cgtcttggct cactgcaacc 6120

tccgcttccc aggttcaagc aaatttcctg cctcagccac ccaagtagct gggattacag 6180

gtgtatgcca ccacgcccgg ctaatttttg tatttttagc agagatgggg ttttcccatg 6240

ttggccaggc tggtcttgaa ctcccgacct caggtgatcc acccacctcg gtctcccaaa 6300

gcgttgggat tacaggcatg agccactgca cccagcctct acacaacttt aaaaaaacag 6360

taacccaatg aagggcagat gagggtagca gcagagccta gaaaagcgcc tctgcagcca 6420

ccaggcaaat gaggagccag aggagaagtg ctccaggcaa aaggaacagc aagacagaga 6480

ccctgaggct ggaaagtccc ctatgtattt gcatccgaat aaaagccagt tttcccagtg 6540

acccacagag cccggcacta tttgtccaca ccaccttctt gatgtcggga tatcccccac 6600

cttgacctca tatcctcctc ccacactctg ctgcagccac aggggcctcc tcacctattt 6660

cagacactcc cacctcaggg cctttgcgct tgctgcccct gctgcttgga gcactctttc 6720

agcagtgatg catgtggctt tttttttttt tttttttttg agacggagtc ttgctctgtc 6780

actcaggctg gagtgcagtg gcatgatctc agctcactgc aagctccacc tcctgggttc 6840

atgccattct cctgcctcag cctcccgagt agctgggact acaggcgtcc gccaccactc 6900

ctggctaatt tttgtatttt taatggagat ggggtttcat tgtgttaatc aggatggtct 6960

cgatctcctg acctcgtgat cctcccgctt cggcctccca aagtactgag attatggaca 7020

tgagccactg cttggctcct gttttaaaat attttttgcg tgggggtttt tccagtgaga 7080

accagaaaac ctgagacaaa ttctaaaaga gctgtaatgt ttgacctctt tacttaaaat 7140

cacaaggctc ccttccctct tttattcttt tccatatagt tatcacctct aacacatgag 7200

ataaatcatg taattggctg ggcgtggtgg cccacgcctg taatcccagc actttgggag 7260

gcaggtggat cacctgaggt caggagtcca agaccagtct ggccaacatg gtgaaacctc 7320

gtctctacta aaaatacaaa aattagctgg gcgtggtggc gggcgcctgt aatcccagct 7380

gctcgggagg ctgaggcagg agaactgctt gaacccagga gatggaggtt tcggtgagcc 7440

aagatcgtgc tactgcactc cagcctgtaa tcccagcact ttaggaggcc gagacgggca 7500

gatcacctga ggttaggagg agttccagac cagcctggct aacatggtga aaccccattt 7560

ttactaaaaa tacaaaaaat taacccgggt gtggtggcgg gtgcctgtaa tcccagctac 7620

ttgagaagct gaggcaggag aatcgcttga acttaggagg cggaggttgc ggtgagccaa 7680

ttgggccact gcactccagg ctgggtgaca gagtgagact ccgtctcaaa aaaaaaaaaa 7740

aaaaaaaaat tcatgtaatc atcttccttg ttagtctctc acactaggat gtcagctcct 7800

gagggcaagg gttttgtggt cttgatcact gctgccctcc agaggctggg ttctcacaga 7860

ggaggtgttt gccaagcgtt tgttgagtgg ctgcctgaca gcacctgcct accctcctcc 7920

ctgcaggaac tcctgcgttg cgagctgtgc ctgggcatca tgggtgggaa accgcgacac 7980

tcactgtact tcattggcta ccaaggtagg cccaccccct cctcactcct cccacccccc 8040

accgcacccc cactcacacc tgcaccccct gcagatgact tcctgctgta cctggaccct 8100

cactactgcc agcccactgt ggatgtcagc caggccgact tccccctgga ggtgagtggg 8160

agccccagtg tgtggttggg gccatggcgg gtgggcagcc cagcctctga gccttcctcg 8220

tctgtctgcc ccagtccttc cactgcacct cgccccgcaa gatggccttt gccaagatgg 8280

acccaagctg taccgtgggc ttctatgctg gagacaggaa ggagtttgag acactctgct 8340

cagagctgac cagggtgagc aagaggaaag ctggagtggg gtgagggggg cggttgggca 8400

gggctggggg tgaaagagga acagaggcct cgagtccagc agagctgggg tgctgcttcc 8460

aggctagggg tcctaacctg ggaaatgtga agaatgtcca cccctgaaaa ggtgggaacg 8520

ccaaagccca ctgttaaggc atcatccctt ggaggcagct gtatccaggg gctcaagtgg 8580

tgggaaccct ccctgtctct tggccctgct gtccgaccag gactctaccc ttatgggggc 8640

gagagatggc caccagccat tctgggcacc caccctgtgg tcactggaag ggaaacgcag 8700

gttttgcagc aaaaatccca ggactgtcag tctttggctg agctgggtca catgctcatg 8760

gctgagccaa tcactgtggc caggggatgg aaggtgctga ttggctgctc tctcctggag 8820

aggtggtcct gagaagcacc tactaaataa acctgggcta aggaatatgg cttggcactg 8880

tttgtggctg accctgtctc cctcttcccg ccaggtcctc agctcctcct cagccacaga 8940

gcggtacccc atgttcaccc tggccgaggg ccatgctcag gaccacagcc tggacgacct 9000

ctgctcccag ctcgcccagc ccacactccg gctccctcgc acagggcggc tcctcagggc 9060

caaacgcccc agctctgagg actttgtgtt tttataaagg gaggggatga ggggaaagat 9120

acaacactat ttattttttt atttatgtca tgtcgggtgt gggatcttga gctctggcag 9180

tgatgatggt acttcctgtt gtcagcccct caagcccagc tgcaaccagt ctggggccat 9240

tcagccaggg acagagccca cagagcccat acacctgtct cccaccagcg gggccctcct 9300

ggcagggtag ggaaggagga ccccgggcac ccccctcagg gcctgactca cgtactgtag 9360

tttgcactgg acgcccgggc cctccctgtc ccaaagcccc cttgggggaa ctgtggctgc 9420

tgggggccaa taaagctgtg taacttga 9448

<210> 5
<211> 1504
<212> DNA
<213> Homo sapiens

<400> 5
tcccggcgcg tccatgaact cagtgtcgcc ggccgccgcg cagtaccgga gcagcagccc 60

ggaggacgcg cgccgccggc ccgaggcccg caggccgcgg ggtcccagag gcccagaccc 120

caacggcctg gggccttccg gagccagcgg ccccgctctt ggctctcccg gggctggccc 180

gagtgagccg gacgaagtgg acaagttcaa ggccaagttc ctgacagcct ggaacaacgt 240

caagtacggt tgggtggtta aaagccggac cagctttagc aagatctcca gcatccacct 300

ctgtggccgc cgctaccgtt tcgagggcga gggtgacata cagcgtttcc agcgggactt 360

tgtgtcccgc ctgtggctca cataccgccg ggacttcccg ccccttcctg ggggctgcct 420

gacctcggac tgtggctggg ggtgcatgtt acgcagcggc cagatgatgc tggcacaggg 480

ccttctgctg catttcctgc ccagagactg gacatggccc gagggcatgg gcctgggccc 540

ccctgagctg tcagggtcag cctctcccag ccggtaccat gggcctgccc gctggatgcc 600

cccacgctgg gcccagggtg cccctgagct ggagcaggaa cgccggcacc ggcagattgt 660

gtcctggttc gccgaccacc cccggccccc ctttggccta caccggctgg tggagcttgg 720

gcagagctca ggcaagaagg caggtgactg gtatggccca tcgctagtgg cacacatcct 780

caggaaagcc gtggagagct gctccgacgt cacccgcctg gtggtgtacg tttctcagga 840

ctgcacagtg tacaaggcgg atgtggcacg cctggtggcc aggccagacc ccacagccga 900

gtggaagtct gtggtcatcc tggtgcccgt gcgactgggt ggcgagactc tcaaccccgt 960

gtatgtgccc tgcgtgaagg aactcctgcg ttgcgagctg tgcctgggca tcatgggtgg 1020

gaaaccgcga cactcactgt acttcattgg ctaccaagat gacttcctgc tgtacctgga 1080

ccctcactac tgccagccca ctgtggatgt cagccaggcc gacttccccc tggagtcctt 1140

ccactgcacc tcgccccgca agatggcctt tgccaagatg gacccaagct gtaccgtggg 1200

cttctatgct ggagacagga aggagtttga gacactctgc tcagagctga ccagggtcct 1260

cagctcctcc tcagccacag agcggtaccc catgttcacc ctggccgagg gccatgctca 1320

ggaccacagc ctggacgacc tctgctccca gctcgcccag cccacactcc ggctccctcg 1380

cacagggcgg ctcctcaggg ccaaacgccc cagctctgag gactttgtgt ttttataaag 1440

ggaggggatg aggggaaaga tacaacacta tttatttttt atttatgtca tgtcgggtgt 1500

gggg 1504

<210> 6
<211> 474
<212> PRT
<213> Homo sapiens

<400> 6

Met Asn Ser Val Ser Pro Ala Ala Ala Gln Tyr Arg Ser Ser Ser Pro
1               5                   10                  15

Glu Asp Ala Arg Arg Arg Pro Glu Ala Arg Arg Pro Arg Gly Pro Arg
            20                  25                  30

Gly Pro Asp Pro Asn Gly Leu Gly Pro Ser Gly Ala Ser Gly Pro Ala
        35                  40                  45

Leu Gly Ser Pro Gly Ala Gly Pro Ser Glu Pro Asp Glu Val Asp Lys
    50                  55                  60

Phe Lys Ala Lys Phe Leu Thr Ala Trp Asn Asn Val Lys Tyr Gly Trp
65                  70                  75                  80

Val Val Lys Ser Arg Thr Ser Phe Ser Lys Ile Ser Ser Ile His Leu
                85                  90                  95

Cys Gly Arg Arg Tyr Arg Phe Glu Gly Glu Gly Asp Ile Gln Arg Phe
            100                 105                 110

Gln Arg Asp Phe Val Ser Arg Leu Trp Leu Thr Tyr Arg Arg Asp Phe
        115                 120                 125

Pro Pro Leu Pro Gly Gly Cys Leu Thr Ser Asp Cys Gly Trp Gly Cys
    130                 135                 140

Met Leu Arg Ser Gly Gln Met Met Leu Ala Gln Gly Leu Leu Leu His
145                 150                 155                 160

Phe Leu Pro Arg Asp Trp Thr Trp Ala Glu Gly Met Gly Leu Gly Pro
                165                 170                 175

Pro Glu Leu Ser Gly Ser Ala Ser Pro Ser Arg Tyr His Gly Pro Ala
            180                 185                 190

Arg Trp Met Pro Pro Arg Trp Ala Gln Gly Ala Pro Glu Leu Glu Gln
        195                 200                 205

Glu Arg Arg His Arg Gln Ile Val Ser Trp Phe Ala Asp His Pro Arg
    210                 215                 220

Ala Pro Phe Gly Leu His Arg Leu Val Glu Leu Gly Gln Ser Ser Gly
225                 230                 235                 240

Lys Lys Ala Gly Asp Trp Tyr Gly Pro Ser Leu Val Ala His Ile Leu
                245                 250                 255

Arg Lys Ala Val Glu Ser Cys Ser Asp Val Thr Arg Leu Val Val Tyr
            260                 265                 270

Val Ser Gln Asp Cys Thr Val Tyr Lys Ala Asp Val Ala Arg Leu Val
        275                 280                 285

Ala Arg Pro Asp Pro Thr Ala Glu Trp Lys Ser Val Val Ile Leu Val
    290                 295                 300

Pro Val Arg Leu Gly Gly Glu Thr Leu Asn Pro Val Tyr Val Pro Cys
305                 310                 315                 320

Val Lys Glu Leu Leu Arg Cys Glu Leu Cys Leu Gly Ile Met Gly Gly
                325                 330                 335

Lys Pro Arg His Ser Leu Tyr Phe Ile Gly Tyr Gln Asp Asp Phe Leu
            340                 345                 350

Leu Tyr Leu Asp Pro His Tyr Cys Gln Pro Thr Val Asp Val Ser Gln
        355                 360                 365

Ala Asp Phe Pro Leu Glu Ser Phe His Cys Thr Ser Pro Arg Lys Met
    370                 375                 380

Ala Phe Ala Lys Met Asp Pro Ser Cys Thr Val Gly Phe Tyr Ala Gly

385                 390                 395                 400

Asp Arg Lys Glu Phe Glu Thr Leu Cys Ser Glu Leu Thr Arg Val Leu
                405                 410                 415

Ser Ser Ser Ser Ala Thr Glu Arg Tyr Pro Met Phe Thr Leu Ala Glu
            420                 425                 430

Gly His Ala Gln Asp His Ser Leu Asp Asp Leu Cys Ser Gln Leu Ala
        435                 440                 445

Gln Pro Thr Leu Arg Leu Pro Arg Thr Gly Arg Leu Leu Arg Ala Lys
    450                 455                 460

Arg Pro Ser Ser Glu Asp Phe Val Phe Leu
465                 470

<210>  7
<211>  2720
<212>  DNA
<213>  Homo sapiens

<400>  7
gcgcagctcc gagggctggc ggctcggggc gccgaggctt ccgcgcccgc tggttcctcg      60

ctccggggcg gagctcgcga tagcgaccgg gagcagggcg cggggcggga cccaggtccg     120

aggcgaggaa gccggaagcc aggcgcgggg agcctccccc ttcgactgca gcctcgctcc     180

gtgccttctg cgcgcctggg atcccggagc ctgcctaggt tctgtgcgct cccgcccagg     240

ccggtgcccg ccgcccgcct gcgccccagg caggtcccag gcctccggct gctcccggcc     300

gaaggtgggg acaggcagtg gcaggcacca ctagcgaggg cgtttgggaa cccagggtga     360

ccacggcgca gccatgggga ccgcgcttgt gtaccatgag gacatgacgg ccacccggct     420

gctctgggac gaccccgagt gcgagatcga gcgtcctgag cgcctgaccg cagccctgga     480

tcgcctgcgg cagcgcggcc tggaacagag gtgtctgcgg ttgtcagccc gcgaggcctc     540

ggaagaggag ctgggcctgg tgcacagccc agagtatgta tccctggtca gggagaccca     600

ggtcctaggc aaggaggagc tgcaggcgct gtccggacag ttcgacgcca tctacttcca     660

cccgagtacc tttcactgcg cgcggctggc cgcaggggct ggactgcagc tggtggacgc     720

tgtgctcact ggagctgtgc aaaatgggct tgccctggtg aggcctcccg ggcaccatgg     780

ccagagggcg gctgccaacg ggttctgtgt gttcaacaac gtggccatag cagctgcaca     840

tgccaagcag aaacacgggc tacacaggat cctcgtcgtg gactgggatg tgcaccatgg     900

ccaggggatc cagtatctct ttgaggatga ccccagcgtc ctttacttct cctggcaccg     960

ctatgagcat gggcgcttct ggcctttcct gcgagagtca gatgcagacg cagtggggcg    1020

gggacagggc ctcggcttca ctgtcaacct gccctggaac caggttggga tgggaaacgc    1080

tgactacgtg gctgccttcc tgcacctgct gctcccactg gcctttgagt ttgaccctga 1140

gctggtgctg gtctcggcag gatttgactc agccatcggg gaccctgagg ggcaaatgca 1200

ggccacgcca gagtgcttcg cccacctcac acagctgctg caggtgctgg ccggcggccg 1260

ggtctgtgcc gtgctggagg gcggctacca cctggagtca ctggcggagt cagtgtgcat 1320

gacagtacag acgctgctgg gtgacccggc cccacccctg tcagggccaa tggcgccatg 1380

tcagagtgcc ctagagtcca tccagagtgc ccgtgctgcc caggccccgc actggaagag 1440

cctccagcag caagatgtga ccgctgtgcc gatgagcccc agcagccact ccccagaggg 1500

gaggcctcca cctctgctgc ctgggggtcc agtgtgtaag gcagctgcat ctgcaccgag 1560

ctccctcctg gaccagccgt gcctctgccc cgcaccctct gtccgcaccg ctgttgccct 1620

gacaacgccg gatatcacat tggttctgcc ccctgacgtc atccaacagg aagcgtcagc 1680

cctgagggag gagacagaag cctgggccag gccacacgag tccctggccc gggaggaggc 1740

cctcactgca cttgggaagc tcctgtacct cttagatggg atgctggatg ggcaggtgaa 1800

cagtggtata gcagccactc cagcctctgc tgcagcagcc accctggatg tggctgttcg 1860

gagaggcctg tcccacggag cccagaggct gctgtgcgtg gccctgggac agctggaccg 1920

gcctccagac ctcgcccatg acgggaggag tctgtggctg aacatcaggg gcaaggaggc 1980

ggctgcccta tccatgttcc atgtctccac gccactgcca gtgatgaccg gtggtttcct 2040

gagctgcatc ttgggcttgg tgctgcccct ggcctatggc ttccagcctg acctggtgct 2100

ggtggcgctg gggcctggcc atggcctgca gggcccccac gctgcactcc tggctgcaat 2160

gcttcggggg ctggcagggg gccgagtcct ggccctcctg gaggagaact ccacacccca 2220

gctagcaggg atcctggccc gggtgctgaa tggagaggca cctcctagcc taggcccttc 2280

ctctgtggcc tccccagagg acgtccaggc cctgatgtac ctgagagggc agctggagcc 2340

tcagtggaag atgttgcagt gccatcctca cctggtggct tgaaatcggc caaggtggga 2400

gcatttacac cgcagaaatg acaccgcacg ccagcgcccc gcggccgcga tccggacccc 2460

aagcccacgg ctccctcgac tctggggcac ggaaccccgc ccactcccaa tccccgcgcc 2520

ccgccctctc ccacccgtgc ttcccccgct ccacccctca cctcacctcg cccccgcccc 2580

acccatcgcg ccccggcccg tcccatcgag gcccatgcaa cccacgctcg gtcccgttcc 2640

ggcccctgcg ccccgctgcg cttcgctccc cgcccttgcg ccgttagtaa acatcgctca 2700

aacgaaaaaa aaaaaaaaaa 2720

<210> 8
<211> 21
<212> DNA
<213> Artificial Sequence

<220>
<223> HSC70 sense siRNA

<400>  8
gaucgauucu cucuaugaat t                                                21


<210>  9
<211>  21
<212>  DNA
<213>  Artificial Sequence

<220>
<223>  HSC70 antisense siRNA

<400>  9
uucauagaga gaaucgauct t                                                21


<210>  10
<211>  21
<212>  DNA
<213>  Artificial Sequence

<220>
<223>  LAMP2A sense siRNA

<400>  10
ccaucagaau uccauugaat t                                                21


<210>  11
<211>  21
<212>  DNA
<213>  Artificial Sequence

<220>
<223>  LAMP2A antisense siRNA

<400>  11
uucaauggaa uucugauggt t                                                21


<210>  12
<211>  22
<212>  DNA
<213>  Artificial Sequence

<220>
<223>  HDAC10 forward PCR primer

<400>  12
atctctttga ggatgacccc ag                                               22


<210>  13
<211>  22
<212>  DNA
<213>  Artificial Sequence

<220>
<223>  HDAC10 reverse PCR primer

<400>  13
actgcgtctg catctgactc tc                                               22


<210>  14

<211>  22
<212>  DNA
<213>  Artificial Sequence

<220>
<223>  HDAC6 forward PCR primer

<400>  14
caaggaacac agttcacctt cg                                                22


<210>  15
<211>  22
<212>  DNA
<213>  Artificial Sequence

<220>
<223>  HDAC6 reverse PCR primer

<400>  15
gttccaaggc acattgatgg ta                                                22


<210>  16
<211>  20
<212>  DNA
<213>  Artificial Sequence

<220>
<223>  HSC70 forward PCR primer

<400>  16
tgctgctgct attgcttacg                                                   20


<210>  17
<211>  24
<212>  DNA
<213>  Artificial Sequence

<220>
<223>  HSC70 reverse PCR primer

<400>  17
tcaatagtga ggattgacac atca                                              24


<210>  18
<211>  20
<212>  DNA
<213>  Artificial Sequence

<220>
<223>  SDHA forward PCR primer

<400>  18
tgggaacaag agggcatctg                                                   20


<210>  19
<211>  22
<212>  DNA
<213>  Artificial Sequence

<220>

<223>  SDHA reverse PCR primer

<400>  19
ccaccactgc atcaaattca tg                                                22


<210>  20
<211>  21
<212>  DNA
<213>  Artificial Sequence

<220>
<223>  HPRT forward PCR primer

<400>  20
tgacactggc aaaacaatgc a                                                 21


<210>  21
<211>  21
<212>  DNA
<213>  Artificial Sequence

<220>
<223>  HPRT reverse PCR primer

<400>  21
ggtccttttc accagcaagc t                                                 21

## Description

The present invention relates to a method of identifying a subj ect being susceptible to a co-treatment with an histone deacetylase 10 (HDAC10)-inhibitor, said subject being in need of and/or being treated with chemotherapy against a cancer, comprising the steps of a) determining the amount of an HDAC10 gene product and/or an autophagy-related gene 4D (ATG4D) product in a cancer sample from said subject, b) comparing the amount of said HDAC10 gene product as determined in step a) with a reference amount for said HDAC10 gene product and/or the amount of ATG4D product as determined in step a) with a reference amount for said ATG4D product, and c) identifying a subject being susceptible to co-treatment with an HDAC10-inhibitor. Moreover, the present invention relates to a method of predicting the risk of recurrence and/or progression of a cancer in an subject treated by chemotherapy, comprising the steps of a) determining the amount of an HDAC10 gene product and/or an ATG4D product in a cancer sample from said subject, b) comparing the amount of said HDAC10 gene product as determined in step a) with a reference amount for said HDAC10 gene product and/or the amount of ATG4D product as determined in step a) with a reference amount for said ATG4D product, and c) predicting the risk of recurrence and/or progression of a cancer treated by chemotherapy. The present invention also relates to a device for identifying a subject being susceptible to co-treatment with an HDAC10-inhibitor, a kit adapted to carry out the method of the present invention. Finally, the invention relates to 2-(4-butoxyphenyl)-N-hydroxyacetamide (bufexamac) for use in treating cancer in a subject, wherein said bufexamac is to be applied in a co-treatment with chemotherapy.

A significant problem in tumor therapy is that many malignant tumors develop frequently resistance against many conventional chemotherapeutics. The resistance is a consequence of an increase in multidrug-resistance of the tumor cells as well a dedifferentiation of the tumor cells. In particular, resistance against chemotherapy is a problem in the therapy of advance stage neuroblastomas.

Histone Deacetylases (HDAC, EC number 3.5.1) are a group of hydrolases removing the acetyl group from an ε-N-acetyl lysine amino acid of a histone. Depending on sequence identity and domain organization, HDACs have been classified into class I (including HDAC1 - 3 and 8), class IIa (HDAC4, 5, 7, 9), class IIb (HDAC6 and 10), class III (including sirtuins) and class IV (HDAC11) (Dokmanovic et al., 2007, Mol Cancer Res October 5; 981-989). It has become clear over recent years that HDACs are not restricted to histones as substrates and that they have functions in several regulative pathways of the cell (Choudhary, C, Kumar, C, Gnad, F, Nielsen, ML, Rehman, M, Walther, TC et al., (2009) Lysine acetylation targets protein complexes and co-regulates major cellular functions. Science 325: 834-40.).

HDAC inhibitors have been traditionally used in psychiatry and neurology as mood stabilizers and anti-epileptics (e.g. valproic acid). More recently, they have been studied for their effects in neurodegenerative diseases, but also in a supportive manner in cancer therapy (Bolden, JE, Peart, MJ and Johnstone, RW, (2006) Anticancer activities of histone deacetylase inhibitors. Nat Rev Drug Discov 5: 769-84.). Despite the fact that several non-histone substrates have been identified for the various HDAC, information is scarce as to which molecular pathways mediate the effects of HDAC inhibitor treatment in cancer; even less is known as to if all cancer patients or just certain cohorts would benefit from HDAC treatment (Witt, O, Deubzer, HE, Milde, T and Oehme, I, (2009) HDAC family: What are the cancer relevant targets? Cancer Lett 277: 8-21.). Moreover, it is unknown, how a potential cohort of patients who would benefit from treatment with a HDAC inhibitor could be identified (Witt, O, Deubzer, HE, Milde, T and Oehme, I, (2009) HDAC family: What are the cancer relevant targets? Cancer Lett 277: 8-21.).

HDAC1, -2, and 8 have been suggested to play a role in reduction of multi-drug resistance, apoptosis, and differentiation of tumor cells (see Oehme 2009, Expert Opin Investig Drugs 18: 1605-1617; Witt 2009, Curr Pharm Des 15:436-447; Keshelva 2007, J Natl Cancer Inst 99: 1107-1119; Oehme 2009, Clin Cancer Res 15: 91-99; Witt 2009, Cancer Lett 277: 8-21). HDAC10 has been merely reported to be involved in deacetylation and thioredoxin regulation (Lai 2010, J Biol Chem 285: 7187-7196; Lee 2010, Mol Cells 30: 107-112).

The technical problems underlying the present invention can be seen as the provision of means and methods for efficiently treating cancer by the embodiments characterized in the claims and herein below.

Accordingly, the present invention relates to a method of identifying a subject being susceptible to a co-treatment with an histone deacetylase 10 (HDAC10)-inhibitor, said subject being in need of or being treated with chemotherapy against a tumor, comprising the steps of:
a) determining the amount of HDAC 10 and/or autophagy-related gene 4D (ATG4D) product in a tumor sample from said subject;
b) comparing the amount of said HDAC10 as determined in step a) with a reference amount for said HDAC10 and/or the amount of ATG4D as determined in step a) with a reference amount for said ATG4D; and
c) identifying a subject being susceptible to co-treatment with an HDAC10-inhibitor.

The method of the present invention allows assessing whether a subject who is in need of treatment by chemotherapy or who is being treated with said therapy is susceptible to a co-treatment with an histone-deacetylase 10 (HDAC10)-inhibitor. Preferably, by carrying out the method of the present invention decisions can be made whether said co-treatment shall be initiated or not.

The method of the current invention, preferably, is an in vitro method. Moreover, it may comprise steps in addition to those explicitly mentioned above. For example, further steps may relate to cell and/or compound pre-treatments or evaluation of the results obtained by the method. The method may be carried out manually or assisted by automation. Preferably, steps a) and/or b) and/or c) or parts thereof may in total or in part be assisted by automation, e.g., by a suitable robotic equipment for the determining the amount of HDAC10 and/or autophagy-related gene 4D (ATG4D) product in step a), or a computer-implemented calculation or comparison step in step b).

The term "identifying" as used herein means assessing whether a subject will be susceptible to a co-treatment with an HDAC10-inhibitor during chemotherapy or not. It is to be understood that a subject who is "susceptible to co-treatment with an HDAC10-inhibitor", preferably, will have a health benefit as a consequence of said co-therapy (such as, e.g. increased survival time, an increased time of disease-free state after treatment, an increased time of progression-free survival, amelioration of any symptons associated with the cancer, reduction of side-effects, reduction of long term health defects) as compared to a subject not receiving said co-treatment, whereas a subject not susceptible to said co-treatment will not have such a health benefit after receiving said co-treatment. As will be understood by the person skilled in the art, such an assessment is usually not intended to be correct for all (i.e. 100%) of the subjects to be identified. The term, however, requires that a statistically significant portion of subjects can be identified (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Preferably, the probability envisaged by the present invention allows that the diagnosis will be correct for at least 60%, at least 70%, at least 80%, or at least 90% of the subj ects of a given cohort or population.

The term "subject" as used herein relates to animals, preferably mammals, and, more preferably, humans. It is, however, envisaged in accordance with the method of the present invention that the subject is in need of treatment and/or being treated with cancer therapy. A subject in need of treatment with cancer therapy is a subject suffering from cancer. It is to be understood that said cancer may be any type of cancer; preferably, said cancer is a neurological or neuroendocrine tumor, more preferably a neuroblastoma, and most preferably a neuroblastoma of International Neuroblastoma Staging System (INSS) stage 4. It is, however, also envisaged by the present specification that the cancer, preferably, is a pediatric embryonal tumor such as, e.g., medulloblastoma, retinoblastoma, atypical teratoid rhabdoid tumor (ATRT), ependymoma, hepatoblastoma, nephroblastoma, a ewing sarcoma family tumor, or any other small round blue cell tumors such as, e.g. melanoma, lung carcinoma, or lymphoma. A subject being treated with cancer therapy is a subject having received or receiving at least one dose of a chemotherapeutic agent as described herein below within a certain time frame before or after the method of the present invention is performed on a sample from said subject. Preferably, said time frame is six months, more preferably two months, or, most preferably, one month.

The term "treatment" refers to all measures conducted to ameliorate the diseases or disorders referred to herein or the symptoms accompanied therewith to a significant extent. Said treatment as used herein also includes an entire restoration of the health, i.e. cure, with respect to the diseases or disorders referred to herein. It is to be understood that treating as used in accordance with the present invention may not be effective in all subjects to be treated. However, the term shall require that a statistically significant portion of subjects suffering from a disease or disorder referred to herein can be successfully treated. Whether a portion is statistically significant can be determined without further ado by the person skilled in the art as described herein above.

The term "histone deacetylase 10-inhibitor" or "HDAC10-inhibitor" relates to a chemical compound inhibiting activity of HDAC 10; the inhibiting activity of a chemical compound on HDAC10 can be assayed as described in the accompanying examples. Preferably, the HDAC10-inhibitor belongs to one of the following chemical classes of hydroxamates, peptides, preferably cyclic tetrapeptides or depsipeptides, benzamides, ketones, and aliphatic acid compounds. More preferably, the HDAC10-inhibitor is a pan-HDAC-inhibitor inhibiting all or most HDACs including HDAC10, like e.g. (2E)-N-hydroxy-3-[4-({[2-(2-methyl-1H-indol-3-yl)ethyl]amino}methyl)phenyl]acrylamide (Panobinostat), 7-[4-(dimethylamino)phenyl]-N-hydroxy-4,6-dimethyl-7-oxohepta-2,4-dienamide (Trichostatin A), N-hydroxy-N'-phenyl-octanediamide (Vorinostat), 3-(dimethylaminomethyl)-N-[2-[4-(hydroxycarbamoyl)phenoxy]ethyl]-1-benzofuran-2-carboxamide (PCI-24781). Even more preferably, the HDAC inhibitor is an inhibitor specific for HDAC class IIb or HDAC10; most preferably, the HDAC inhibitor is 2-(4-butoxyphenyl)-N-hydroxyacetamide (bufexamac).

The term "cancer therapy", as used herein, relates to the treatment of cancer by application to a patient of a chemical substance known to inhibit growth of cancer cells, to kill cancer cells, or to cause the body of a patient to inhibit the growth of or to kill cancer cells. Preferably, cancer therapy is chemotherapy, targeted therapy, immunotherapy, or any combination thereof. It is, however, also envisaged that the cancer therapy is radiation therapy, alone or combination with other therapy regimens.

As used herein, the term "chemotherapy" relates to treatment of a subject with an antineoplastic drug. Preferably, chemotherapy is a treatment including alkylating agents (e.g. cyclophosphamide), platinum (e.g. carboplatin), anthracyclines (e.g. doxorubicin, epirubicin, idarubicin, or daunorubicin) and topoisomerase II inhibitors (e.g. etoposide, irinotecan, topotecan, camptothecin, or VP16), anaplastic lymphoma kinase (ALK)-inhibitors (e.g. Crizotinib or AP26130), aurora kinase inhibitors (e.g. N-[4-[4-(4-Methylpiperazin-1-yl)-6-[(5-methyl-1H-pyrazol-3-yl)amino]pyrimidin-2-yl]sulfanylphenyl]cyclopropanecarboxamide (VX-680)), antiangiogenic agents (e.g. Bevacizumab), or Iodine131-1-(3-iodobenzyl)guanidine (therapeutic metaiodobenzylguanidine), HDAC8-Inhibitors, alone or any suitable combination thereof. It is to be understood that chemotherapy relates to a complete cycle of treatment, i.e. a series of several (e.g. four, six, or eight) doses of antineoplastic drug or drugs applied to a subject separated by several days or weeks without such application.

The term "targeted therapy", as used herein, relates to application to a patient a chemical substance known to block growth of cancer cells by interfering with specific molecules known to be necessary for tumorigenesis or cancer or cancer cell growth. Examples known to the skilled artisan are small molecules like, e.g. Bcl-2-Inhibitors (e.g. Obatoclax) and PARP-inhibitors (e.g. Iniparib), or monoclonal antibodies like, e.g., Rituximab or Trastuzumab.

The term "immunotherapy" as used herein relates to the treatment of cancer by modulation of the immune response of a subject. Said modulation may be inducing, enhancing, or suppressing said immune response.

The term "radiation therapy" or "radiotherapy" is known to the skilled artisan. The term relates to the use of ionizing radiation to treat or control cancer.

The term "co-treatment" as used herein relates to applying to a subject a therapeutically effective amount of an HDAC10-inhibitor as described herein below concomitantly to the cancer therapy of the present invention, "concomitant" relating to treatment (cancer therapy) and co-treatment being temporally connected. Preferably, co-treatment is applied shortly, e.g. less than eight, six, four, three, two, or one week(s) before or after cancer therapy. More preferably, cancer therapy and co-treatment are applied during the same time. Most preferably, cancer therapy and co-treatment are applied simultaneously. It is, however, also envisaged by the current invention that a therapeutically effective amount of an HDAC10-inhibitor is applied to a subject several months or years before cancer therapy or as single therapy.

As used herein, the term "gene product" relates to a product which is generated during the gene expression process. The mechanisms of gene expression are well-known to the one skilled in the art to include the basic mechanisms of transcription, i.e. formation of RNA corresponding to the said gene or parts thereof, and translation, i.e. production of polypeptide molecules having an amino acid sequence encoded by said RNA according to the genetic code; it is well-known to the one skilled in the art that other cellular processes may be involved in gene expression as well, e.g. RNA processing, RNA editing, proteolytic processing, protein editing, and the like. The term gene product, thus, includes RNA, preferably mRNA, as well as the translated polypeptides. It is clear from the above that the term gene product also includes fragments of said RNA(s), preferably with a length of at least ten, at least twelve, at least 20, at least 50, or at least 100 nucleotides, and fragments (peptides) from said polypeptides, preferably with a length of at least eight, at least ten, at least twelve, at least 15, at least 20 amino acids.

The term "HDAC10 gene" relates to the human HDAC10 gene (SEQ ID NO: 1, Genbank Acc No. NG_029758.1 GI:343780922). Main products of this gene are the HDAC10 mRNA (comprising the sequence as in SEQ ID NO: 2, Genbank Acc No. BC094734.1 GI:66267380), the HDAC10 mRNA transcript variant 1 (comprising the sequence as in SEQ ID NO: 7, Genbank Acc No. NM_032019.5 GI:226529782), and the HDAC10 polypeptide (comprising the sequence as in SEQ ID NO: 3, Genbank Acc No. Q969S8.1 GI:27734403).

The term " autophagy-related gene 4D" or "ATG4D" relates to the autophagy-related gene 4D (SEQ ID NO: 4, Genbank Acc No. NC_000019.9 GI:224589810). Main products of this gene are the ATG4D mRNA (comprising the sequence as in SEQ ID NO: 5, Genbank Acc No. AJ312332.1 GI:27763974) and the ATG4D polypeptide (comprising the sequence as in SEQ ID NO: 6, Genbank Acc No. Q86TL0.1 GI:61211809).

Moreover, the term "gene product" as used in accordance with the present invention further encompasses variants of the aforementioned specific polynucleotides. Said variants may represent orthologs, paralogs or other homologs of the polynucleotide of the present invention. The polynucleotide variants, preferably, comprise a nucleic acid sequence characterized in that the sequence can be derived from the aforementioned specific nucleic acid sequences shown in SEQ ID NOs: 1, 2, 4, or 5 by at least one nucleotide substitution, addition and/or deletion whereby the variant nucleic acid sequence shall still encode a polypeptide having the biological activity and/or immunological properties of the HDAC10 or ATG4D protein as specified above. Variants also encompass polynucleotides comprising a nucleic acid sequence which is capable of hybridizing to the aforementioned specific nucleic acid sequences, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6 x sodium chloride/sodium citrate (= SSC) at approximately 45°C, followed by one or more wash steps in 0.2 x SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5 × SSC (pH 7.2). If organic solvent is present in the abovementioned buffer, for example 50% formamide, the temperature under standard conditions is approximately 42°C. The hybridization conditions for DNA:DNA hybrids are preferably for example 0.1 × SSC and 20°C to 45°C, preferably between 30°C and 45°C. The hybridization conditions for DNA:RNA hybrids are preferably, for example, 0.1 × SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. Alternatively, polynucleotide variants are obtainable by PCR-based techniques such as mixed oligonucleotide primer- based amplification of DNA, i.e. using degenerated primers against conserved domains of the polypeptides of the present invention. Conserved domains of the polypeptide of the present invention may be identified by a sequence comparison of the nucleic acid sequence of the polynucleotide or the amino acid sequence of the polypeptide of the present invention with sequences of other HDAC10 polypeptides. Oligonucleotides suitable as PCR primers as well as suitable PCR conditions are described in the accompanying Examples. As a template, DNA or cDNA from bacteria, fungi, plants or animals may be used. Further, variants include polynucleotides comprising nucleic acid sequences which are at least 70%, at least 75%, at least 80%, at least 85%, at least 90%, at least 95%, at least 98% or at least 99% identical to the nucleic acid sequences shown in SEQ ID NOs: 1, 2, 4, or 5. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the amino acid sequences shown in SEQ ID NOs: 3 or 6. It will be understood that all of the aforementioned variants shall still encode polypeptides or shall still be polypeptides having the biological activity and/ or immunological properties of HDAC10 or ATG4D, respectively. The percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit [Needleman and Wunsch (J. Mol. Biol. 48; 443-453 (1970)) and Smith and Waterman (Adv. Appl. Math. 2; 482-489 (1981))], which are part of the GCG software packet [Genetics Computer Group, 575 Science Drive, Madison, Wisconsin, USA 53711 (1991)], are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

The polynucleotide, preferably, is DNA including cDNA or RNA. The term encompasses single as well as double stranded polynucleotides. Moreover, comprised are also chemically modified polynucleotides including naturally occurring modified polynucleotides such as glycosylated or methylated polynucleotides or artificial modified one such as biotinylated polynucleotides.

Preferably, the term "polypeptide" refers to the polypeptides comprising the sequences as described herein above and variants of said specific polypeptides which include allelic variants, orthologs and homologs. Orthologs of human said polypeptides in other organisms, e.g., in rodents are also well known in the art.

Determining the amount of HDAC10 gene product and/or autophagy-related gene 4D (ATG4D) product relates to measuring the amount of said HDAC10 and/or ATG4D gene product, preferably semi-quantitatively or quantitatively. Measuring can be done directly or indirectly. Preferably, measuring is performed on a processed sample, said processing comprising extraction of polynucleotides or polypeptides from the sample. More preferably, the amount of HDAC10 gene product and/or autophagy-related gene 4D (ATG4D) product is determined on a tissue section from said sample.

The amount of the polynucleotides of the present invention can be determined with several methods well-known in the art. Quantification preferably is absolute, i.e. relating to a specific number of polynucleotides or, more preferably, relative, i.e. measured in arbitrary normalized units. Preferably, a normalization is carried out by calculating the ratio of a number of specific polynucleotides and total number of polynucleotides or a reference amplification product comprised by a sample as set forth elsewhere herein in detail. Methods allowing for absolute or relative quantification are well known in the art. E.g., quantitative PCR methods are methods for relative quantification; if a calibration curve is incorporated in such an assay, the relative quantification can be used to obtain an absolute quantification. Other methods known are, e.g. nucleic acid sequence-based amplification (NASBA) or the Branched DNA Signal Amplification Assay method in combination with dot blot or luminex detection of amplified polynucleotides.

Preferably, the polynucleotide amounts are normalized polynucleotide amounts, i.e. the polynucleotide amounts obtained are set into relation to at least one reference amplification product, thereby, preferably, setting the polynucleotide amounts into relation to the number of cells in the sample and/or the efficiency of polynucleotide amplification. Thus, preferably, the reference amplification product is a product obtained from a polynucleotide known to have a constant abundancy in each cell, i.e. a polynucleotide comprised in most, preferably all, cells of a sample in approximately the same amount. More preferably, the reference amplification product is amplified from a chromosomal or mitochondrial gene or from the mRNA of a housekeeping gene.

The amount of peptides or polypeptides of the present invention can be determined in various ways. Direct measuring relates to measuring the amount of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay and / or immunohistochemistry devices and methods which may utilize labeled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays, Cobalt Binding Assays, and latex agglutination assays.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Nonspecific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Suitable methods are described in the following.

First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable label prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labelling may be done by direct or indirect methods. Direct labelling involves coupling of the label directly (covalently or non-covalently) to the ligand. Indirect labelling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate), CDP-Star™ (Amersham Biosciences), ECF™ (Amersham Biosciences). A suitable enzyme-substrate combination may result in a colored reaction product, fluorescence or chemo luminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include 35S, 125I, 32P, 33P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme- linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

In another preferred embodiment of the present invention, the amount of HDAC10 polypeptide is determined using an enzymatic assay detecting the activity of HDAC10 as described in the accompanying Examples, below.

The term "cancer sample" refers to a sample of a body fluid comprising cancer cells, to a sample of separated cancer cells or to a sample from a tissue or an organ or to a sample of wash/rinse fluid obtained from an outer or inner body surface comprising cancer cells. The sample comprises polynucleotides and/or polypeptides, preferably the sample comprises RNA. Samples can be obtained by well known techniques and include, preferably, scrapes, swabs or biopsies. Such samples can be obtained by use of brushes, (cotton) swabs, spatula, rinse/wash fluids, punch biopsy devices, puncture of cavities with needles or surgical instrumentation. Cancer samples may be obtained from any tissue or organ by, e.g., biopsy or other surgical procedures. Separated cells may be obtained from the body fluids or the tissues or organs by separating techniques such as filtration, centrifugation or cell sorting. It is to be understood that the sample may be further processed in order to carry out the method of the present invention. Particularly, polynucleotides such as DNA or RNA, preferably RNA or cDNA, and/or polypeptides might be obtained from the sample by methods and means known in the art. Thus, the term sample also may relate to polynucleotides, preferably RNA or cDNA, or polypeptides obtained from any sample as mentioned to above.

The term "comparing" as used herein encompasses comparing the amount of gene product determined in step a) of the methods of the present invention to a suitable reference amount, the source of which is specified elsewhere in this description. It is to be understood that comparing as used herein refers to a comparison of amounts. The comparison referred to in step b) of the methods of the present invention may be carried out manually or computer-assisted. For a computer-assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount of gene product to a reference amount it is possible to identify, in a group of subjects being in need of treatment and/or being treated with cancer therapy against a cancer, a subject being susceptible to co-treatment with an HDAC10-inhibitor.

Accordingly, the term "reference", "reference amount" or "reference value" as used herein, preferably, refers to an amount or value which allows differentiation between a subject being susceptible to co-treatment with an HDAC10-inhibitor and a subject not being susceptible to co-treatment with an HDAC10-inhibitor. Accordingly, the reference may be derived as described in the accompanying examples, below. It is to be understood that a reference amount, preferably, is specific for a given gene product, i.e. different gene products may necessitate the use of different reference amounts.

It is also to be understood that the value of the reference or threshold may vary depending on the nature of the gene product (transcript or polypeptide) and depend on how the amount of a gene product is determined in the sample. For example, if the determination of the amount of the gene product includes amplification of the gene product by PCR (polymerase chain reaction), the determined amount of a gene product may depend, e.g., on the oligonucleotides used for the PCR reaction since the amplification efficiency of various oligonucleotide pairs for the amplification of a specific gene product varies. However, the person skilled in the art considers this when calculating the reference amount. Particularly, the person skilled knows that, preferably, the same means and methods have to be used for determining the amounts of a specific gene product in a reference sample and in a test sample.

A reference, reference amount or reference value as referred to herein can, in principle, be calculated for a cohort of subjects as specified above based on the average or mean values for a given peptide or polypeptide by applying standard methods of statistics. In particular, accuracy of a test such as a method aiming to diagnose an event, or not, is best described by its receiver-operating characteristics (ROC) (see, e.g., Zweig 1993, Clin. Chem. 39:561-577). The ROC graph plots all of the sensitivity versus specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC graph indicates the overlap between the two distributions by plotting the sensitivity versus 1-specificity for the complete range of thresholds suitable for making a distinction. On the γ-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC graph is independent of the prevalence of the event in the cohort. Each point on the ROC graph represents a sensitivity/-specificity pair corresponding to a particular decision threshold. A test with perfect discrimination (no overlap in the two distributions of results) has an ROC graph that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC graph falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects being susceptible to the cotreatment, preferably, by establishing a ROC for said cohort as described above and deriving a threshold amount therefrom. Dependent on a desired sensitivity and specificity for a diagnostic method, the ROC plot allows deriving suitable thresholds. It will be understood that an optimal sensitivity is desired for excluding a subject for being for cotreatment (i.e. a rule out) whereas an optimal specificity is envisaged for a subject to be assessed as being susceptible for cotreatment (i.e. a rule in).

Preferably, signal intensity is determined in samples from a cohort of subjects diagnosed with high-risk INSS stage 4 neuroblastoma, and the reference, reference amount or reference value is chosen at the 25th percentile. More preferably, the reference, reference amount or reference value is chosen as described in the examples herein below.

Preferably, an amount of gene product in the cancer sample higher than the reference amount indicates a subj ect susceptible to co-treatment with an HDAC10- inhibitor. More preferably an amount of gene product in the cancer sample significantly higher than the reference amount indicates a subject susceptible to co-treatment with an HDAC10-inhibitor. Most preferably, an amount of gene product in the cancer sample that is statistically significantly higher than the reference amount a subject susceptible to co-treatment with an HDAC10- inhibitor.

Preferably, an amount of gene product in the cancer sample lower than the reference amount indicates a subject not susceptible to co-treatment with an HDAC10- inhibitor. More preferably, an amount of gene product in the cancer sample significantly lower than the reference amount indicates a subject not susceptible to co-treatment with an HDAC10-inhibitor. Most preferably, said amount of gene product in the cancer sample statistically significantly lower than the reference amount indicates a subject not susceptible to co-treatment with an HDAC10- inhibitor.

Particularly, an amount statistically significantly higher (or lower) than a reference amount is an amount which is considered to be significant for the differentiation referred to herein. Whether an amount is statistically significantly higher (or lower) can be determined without further ado by the person skilled in the art using various well known statistic evaluation tools. Preferably, the reference amount is selected such that less than 30%, less than 25%, less than 20%, less than 15%, less than 10%, less than 7%, less than 5%, less than 3%, less than 1%, or less than 0.5% of subjects susceptible to co-treatment with an HDAC10- inhibitor are diagnosed not to be susceptible to co-treatment with an HDAC10-inhibitor and such that less than 20%, less than 15%, less than 10%, less than 7%, less than 5%, less than 3%, less than 1%, or less than 0.5% of subjects not susceptible to co-treatment with an HDAC10- inhibitor are diagnosed to be susceptible to co-treatment with an HDAC10- inhibitor.

Thus, the method of the present invention is particularly advantageous since it allows for rapid, reliable, inexpensive differentiation between subjects susceptible to co-treatment with an HDAC10- inhibitor and subjects not susceptible to co-treatment with an HDAC10-inhibitor. Without applying the method of the present invention, the said subjects not susceptible to co-treatment with an HDAC10- inhibitor might have been examined and treated too much (resulting in an increased risk of adverse side effects and increased health care cost).

The definitions made above apply *mutatis mutandis* to the following embodiments except as stated otherwise:
In another preferred embodiment, the present invention relates to a method of predicting the risk of recurrence and/or progression of a cancer in a subject treated by cancer therapy,
comprising the steps of:
   a) determining the amount of an HDAC10 gene product and/or an ATG4D product in a cancer sample from said subject,
   b) comparing the amount of said HDAC10 gene product as determined in step a) with a reference amount for said HDAC10 gene product and/or the amount of ATG4D product as determined in step a) with a reference amount for said ATG4D product, and
   c) predicting the risk of recurrence and/or progression of a cancer treated by cancer therapy.

As used herein, the term "predicting the risk" relates to providing an estimate for the probability that a subject treated with cancer therapy against cancer will suffer from progressing cancer or recurrence of cancer after said treatment within a certain time window (prediction window). Preferred prediction windows are up to two months, up to six months, up to one year, up to two years, or up to five years. It is to be understood that such an assessment is usually not intended to be correct for all (i.e. 100%) of the subjects. The term, however, requires that said prediction be true for a statistically significant portion of subjects. Whether a portion is statistically significant can be determined without further ado by the person skilled in the art using various well known statistic evaluation tools, e.g. as described herein above. It is also to be understood that the reference values used in step b) of said method must not necessarily be of the same numerical value as the reference values of step b) of the method described herein above. The methods used to determine the reference values are known to the skilled person, e.g. as described herein, e.g. in the accompanying Examples below.

The term "predicting the risk of recurrence and/or progression of a cancer in a subject treated by cancer therapy", preferably, relates a response prediction, i.e. to providing an estimate for the probability that a subject treated with cancer therapy will respond to said therapy.

In another preferred embodiment, the present invention relates to a device for identifying a subject being susceptible to co-treatment with an HDAC10-inhibitor, comprising
a) an analyzing unit for determining the amount of an HDAC10 gene product and/or ATG4D product in a cancer sample from a subject in need of and/or treated by cancer therapy against a cancer; and
b) an evaluation unit for comparing the amount determined by said analyzing unit to a reference amount for said HDAC10 gene product and/or ATG4D product, whereby a subject being susceptible to co-treatment with an HDAC10-inhibitor is identified.

The term "device" as used herein relates to a system of means comprising at least the aforementioned means operatively linked to each other as to allow the identification. Preferred means for determining the amounts of the gene products of the present invention, and means for carrying out the comparison are disclosed above in connection with the method of the invention. Means for determining the amount of a peptide or polypeptide referred to herein are, preferably, detection agents which specifically bind to the said peptide or polypeptides such as antibodies, aptamers or other agents, and which can be detected, e.g., due to the presence of a detectable label upon binding. How to link the means in an operating manner will depend on the type of means included into the device. For example, where means for automatically determining the amount of a peptides are applied, the data obtained by said automatically operating means can be processed by, e.g., a computer program in order to obtain the desired results. Preferably, the means are comprised by a single device in such a case. Said device may accordingly include an analyzing unit for the measurement of the amount of the polynucleotides, peptides or polypeptides in an applied sample and a computer unit for processing the resulting data for the evaluation. Alternatively, where means such as test stripes are used for determining the amount of e.g. peptides or polypeptides, the means for comparison may comprise control stripes or tables allocating the determined amount to a reference amount. The test stripes are, preferably, coupled to a ligand which specifically binds to the peptides or polypeptides referred to herein. The strip or device, preferably, comprises means for detection of the binding of said peptides or polypeptides to the said ligand. Preferred means for detection are disclosed in connection with embodiments relating to the method of the invention above. In such a case, the means are operatively linked in that the user of the system brings together the result of the determination of the amount and the diagnostic or prognostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of raw data which need interpretation by the clinician. Preferably, the output of the device is, however, processed, i.e. evaluated, raw data the interpretation of which does not require a clinician. Further preferred devices comprise the analyzing units/devices (e.g., biosensors, arrays, solid supports coupled to ligands specifically recognizing the peptide, Plasmon surface resonance devices, NMR spectrometers, mass-spectrometers etc.) or evaluation units/devices referred to above in accordance with the method of the invention.

Encompassed by the present invention is also a kit adapted to carry out a method of the present invention, said kit comprising instructions for carrying out the said method, and means for determining the amount of an HDAC10 gene product and/or ATG4D product in a sample from a subject in need of cancer therapy against a neurological tumor, and means for comparing the amount determined by said means to a reference amount for said HDAC10 gene product and/or ATG4D product allowing identifying a subject being susceptible to co-treatment with an HDAC10-inhibitor.

The term "kit" as used herein refers to a collection of the aforementioned means, e.g., a composition comprising the probe oligonucleotides of the current invention and/or means for contacting a sample under conditions which allow for amplification of polynucleotides and for determining the polynucleotides of the present invention based on the amplified polynucleotides, preferably, provided separately or within a single container. The container, also preferably, comprises instructions for carrying out the method of the present invention. The components of the kit are provided, preferably, in a "ready-to-use" manner, e.g., concentrations are adjusted accordingly, etc. Means for determining the amount of a peptide or polypeptide referred to herein are, preferably, detection agents which specifically bind to the said peptide or polypeptides such as antibodies, aptamers or other agents, and which can be detected, e.g., due to the presence of a detectable label upon binding.

The present invention also relates to 2-(4-butoxyphenyl)-N-hydroxyacetamide (bufexamac) for use in treating cancer in a subject suffering therefrom, (i) wherein a sample from said cancer was determined to comprise a high amount of HDAC10 gene product as compared to a reference amount for said HDAC10 gene product and/or a high amount of ATG4D product as compared to a reference amount for said ATG4D product and (ii) wherein said bufexamac is to be applied in a co-treatment with a chemotherapeutic agent.

Preferably, bufexamac is used in a pharmaceutical composition. A "pharmaceutical composition" as used herein comprises the bufexamac of the present invention, optionally one or more chemotherapeutic agent, and optionally one or more pharmaceutically acceptable carrier. It is to be understood, however, that the pharmaceutical composition may consist only of bufexamac in a pharmaceutically acceptable dosage form. The compounds of the present invention, i.e. bufexamac and, optionally, chemotherapeutic agents, can be formulated as pharmaceutically acceptable salts. Acceptable salts comprise acetate, methylester, HCl, sulfate, chloride and the like. The pharmaceutical compositions are, preferably, administered topically or systemically. Suitable routes of administration conventionally used for drug administration are oral, intravenous, or parenteral administration as well as inhalation. However, depending on the nature and mode of action of a compound, the pharmaceutical compositions may be administered by other routes as well.

Moreover, the compounds can be administered in combination with other drugs either in a common pharmaceutical composition or as separated pharmaceutical compositions wherein said separated pharmaceutical compositions may be provided in form of a kit of parts.

The compounds are, preferably, administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

The carrier(s) must be acceptable in the sense of being compatible with the other ingredients of the formulation and being not deleterious to the recipient thereof. The pharmaceutical carrier employed may be, for example, either a solid, a gel or a liquid. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania

The diluent(s) is/are selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like.

A therapeutically effective dose refers to an amount of the compounds to be used in a pharmaceutical composition of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. A typical dose can be, for example, in the range of 10 µg to 1000 mg; however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment. However, depending on the subject and the mode of administration, the quantity of substance administration may vary over a wide range to provide from about 0.01 mg per kg body mass to about 100 mg per kg body mass, preferably.

The pharmaceutical compositions and formulations referred to herein are administered at least once in order to treat or ameliorate or prevent a disease or condition recited in this specification. However, the said pharmaceutical compositions may be administered more than one time, for example from one to four times daily up to a non-limited number of days.

Specific pharmaceutical compositions are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent, or enclosed or encapsulated in a capsule, sachet, cachet, paper or other suitable containers or vehicles. The resulting formulations are to be adopted to the mode of administration, i.e. in the forms of tablets, capsules, suppositories, solutions, suspensions or the like. Dosage recommendations shall be indicated in the prescribers or users instructions in order to anticipate dose adjustments depending on the considered recipient.

All references cited in this specification are herewith incorporated by reference with respect to their entire disclosure content and the disclosure content specifically mentioned in this specification.

### FIGURES

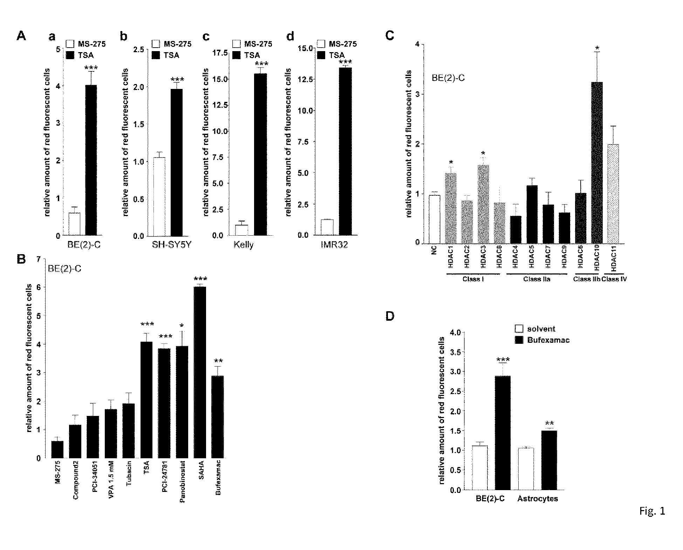

**Figure 1****: HDAC10 inhibition induces accumulation of acidic lysosomes in several neuroblastoma cell lines**
   (A) Accumulation of acidic lysosomes in different NB cell lines. Treatment of BE(2)-C (a), SH-SY5Y (b), Kelly (c) and IMR32 (d) cells with 75 nM pan-HDAC inhibitor TSA (black bar) increased the amount of acridine orange red-fluorescent cells, indicative for acidic lysosomes due to a shift from green to red fluorescence, detected by FACS. Treatment of cells with class I selective inhibitor MS-275 (entinostat, 250 nM; open bar) did not increase the relative amount of red fluorescent cells. Numbers are expressed relative to solvent treated cells. (B) Accumulation of acidic lysosomes by a panel of HDAC inhibitors. BE(2)-C cells treated with a panel of different HDAC inhibitors showed a significant increase in red fluorescence (acridine orange red fluorescent cells) only when treated with the pan-HDACi TSA (75 nM), PCI-24781 (0.5 µM), Panobinostat (100 nM) and SAHA (vorinostat, 0.5 µM) as well as with the class IIb-selective inhibitor bufexamac (100 µM). Treatment with HDAC selective inhibitors Compound2 (40 µM), tubacin (5 µM) and VPA (1.5 mM) did not increase the amount of red fluorescent cells. Numbers are expressed relative to solvent treated cells. Asterisks: one sample t-test, to test whether column means are significantly different from 1.0 (= solvent treatment), * P < 0.05; ** P < 0.01; *** P < 0.001. Error bars represent S.E.M. (C) Accumulation of acidic lysosomes upon RNAi-mediated specific knockdown of HDAC1-11. BE(2)-C cells transiently transfected with siRNAs targeting all classical HDACs (1-11). Numbers are expressed relative to negative control siRNA (NC) transfected cells. Asterisks: one sample t-test, to test whether column means are significantly different from 1.0 (= NC siRNA), * P < 0.05. Error bars represent S.E.M. (D) Lack of accumulation of acidic lysosomes in non-transformed cells. BE(2)-C cells and astrocytes treated with class IIb-selective inhibitor bufexmac. Numbers are expressed relative to solvent treated cells. Asterisks: unpaired two-tailed t-test bufexamac versus solvent, ** P < 0.01; *** P < 0.001.

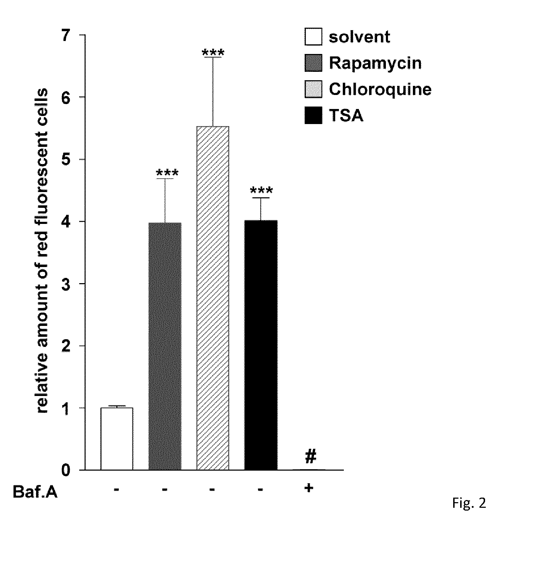

**Figure 2****: Detection of acidic lysosomes in BE(2)-C cells**
   Chloroquine (CQ, 25 µM) and rapamycin (100 nM) (hatched bars) served as positive controls for the induction of acidic lysosomes. BE(2)-C cells treated with TSA (75 nM; black bar) displayed an increase of acridine orange stained red fluorescent cells due to low pH. Co-treatment with Bafilomycin A1 (Baf.A, 10 nM) blocked lysosomal pumps and therefore inhibited the TSA-induced shift to red fluorescence. Numbers are expressed relative to solvent treated cells. Asterisks: unpaired two-tailed t-test TSA versus solvent, *** P < 0.001. Error bars represent S.E.M.

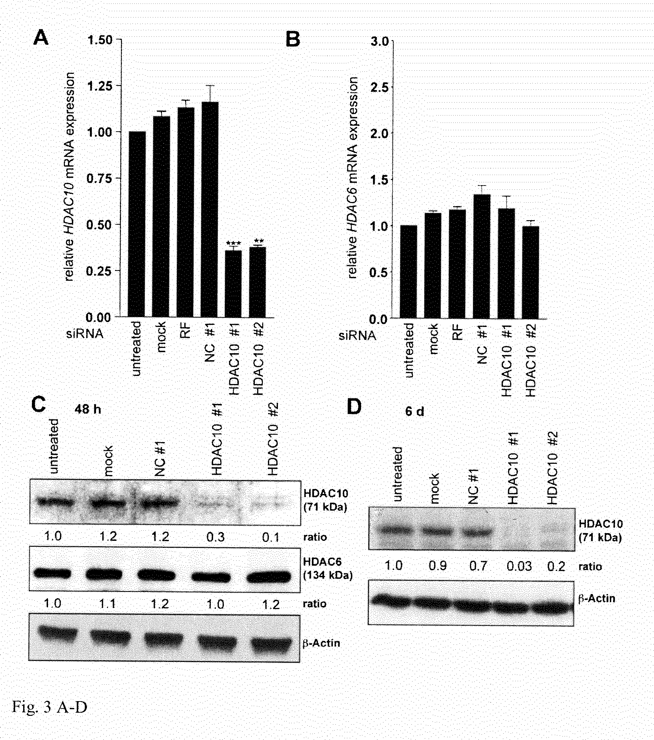

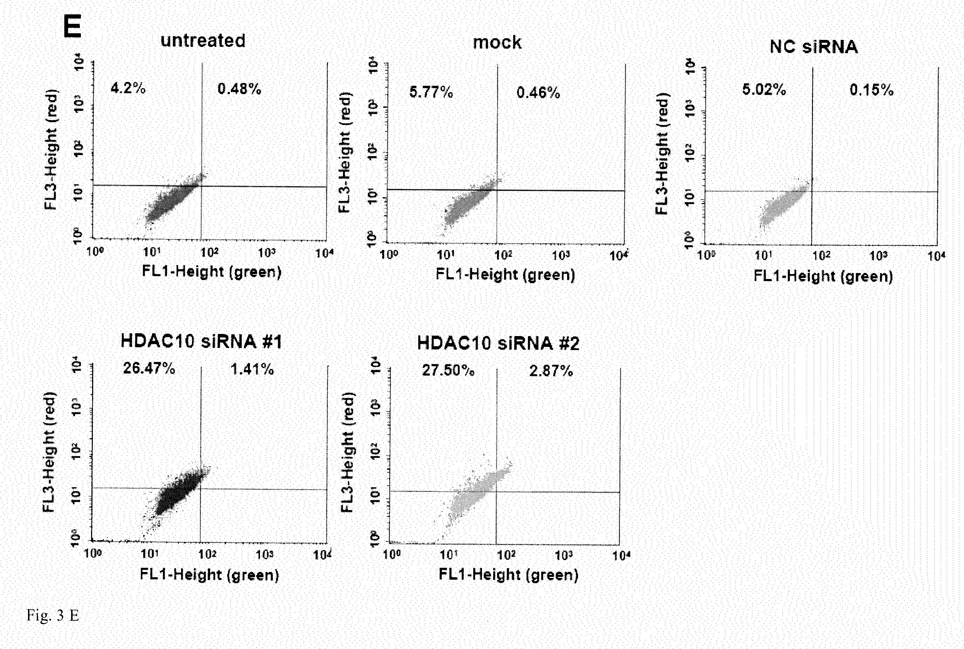

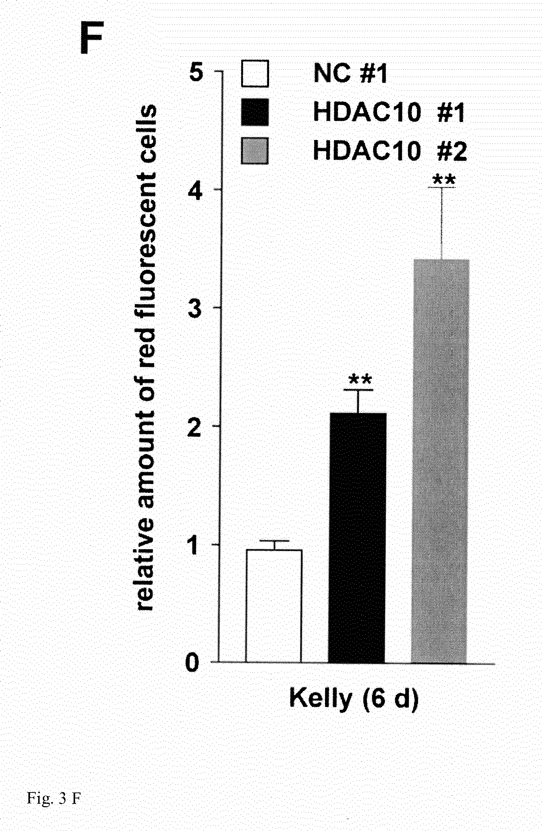

**Figure 3****: Knockdown of *HDAC10* expression in BE(2)-C cells**
   (A) Knockdown of *HDAC10* expression. BE(2)-C cells transfected with siRNA #1 and #2 against *HDAC10* displayed a significant decrease of *HDAC10* mRNA expression in comparison with RISC-free (RF) or negative control (NC) transfected cells (***P<0.001 and **P<0.01). Expression levels were measured with real time RT-PCR and calculated relative to untreated cells. (B) *HDAC6* expression BE(2)-C cells transfected with siRNA #1 and #2 against *HDAC10* displayed no significant changes of HDAC6 mRNA expression in comparison with RISC-free (RF) or negative control (NC) transfected cells. Expression levels were measured with real time RT-PCR 48 h after transfection and calculated relative to untreated cells. (C) Western blot demonstrating the knockdown of HDAC10 protein levels 48 h after transfection of BE(2)-C cells with siRNA #1 and #2 targeting *HDAC10.* HDAC6 protein levels were unaffected by *HDAC10* knockdown. β-Actin protein levels served as loading controls. Untreated = BE(2)-C cells cultured in medium only, mock = BE(2)-C cells incubated with transfection reagent only. Numbers indicate the ratio of HDAC10 or HDAC6 protein levels, respectively, to β-Actin protein levels. (D) Western Blot showing the knockdown of HDAC10 protein levels 6 d after transfection of BE(2)-C cells with two siRNAs targeting *HDAC10* (HDAC10 #1 and #2). β-Actin protein levels served as loading controls. Untreated = BE(2)-C cells cultured in medium only, mock = BE(2)-C cells incubated with transfection reagent only. Numbers indicate the ratio of HDAC10 protein levels to β-Actin protein levels. (E) Scatter Blot showing FL1-Fluorescence (green) and FL3-Fluorescence (red) of acridine orange stained cells. *HDAC10* knockdown (HDAC10 siRNA #1 and #2) induced a shift from green-to-red fluorescence 72 h after transfection. Untreated = BE(2)-C cells cultured in medium only, mock = cells treated with transfection reagent only, NC siRNA = cells transfected with negative control siRNA. (F) Accumulation of acididc lysosomes 6 d post transfection. Kelly cells transfected with two specific siRNAs targeting *HDAC10* (HDAC10 #1 and #2) displayed an increase in red fluorescence in comparison with negative control siRNA (NC #1) transfected cells. Acridine orange red-fluorescent cells detected by FACS are represented in relation to NC transfected cells. Asterisks: unpaired two-tailed t-test *HDAC10* siRNA versus NC siRNA, ** P < 0.01. Error bars represent S.E.M.

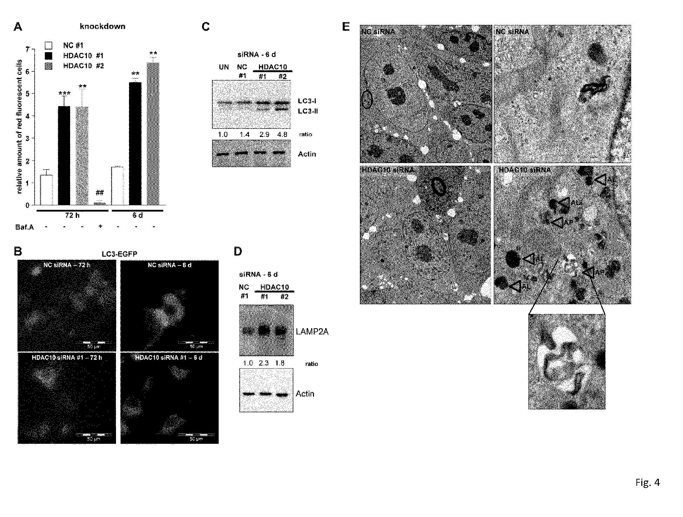

**Figure 4****: Targeting of HDAC10 promotes accumulation of autophagosomes and lysosomes**
   (A) Accumulation of acididc lysosomes 72 h and 6 d post transfection. Cells transfected with two specific siRNAs targeting *HDAC10* (HDAC10 #1 and #2) displayed an increase in red fluorescence in comparison with negative control siRNA (NC #1) transfected cells. Bafilomycin A1 (Baf. A, 10 nM) blocked lysosome acidification induced by *HDAC10* knockdown. Acridine orange red-fluorescent cells detected by FACS are represented in relation to untreated cells. Asterisks: unpaired two-tailed t-test *HDAC10* siRNA versus NC siRNA, ** P < 0.01; *** P < 0.001. Number sign: unpaired two-tailed t-test *HDAC10* siRNA versus *HDAC10* siRNA with Baf. A, ## P < 0.01. Error bars represent S.E.M. (B) Autophagosome formation. Representative pictures of EGFP-LC3 fluorescence (green) in BE(2)-C cells transiently transfected with EGFP-LC3 expressing plasmid following transfection with siRNAs against *HDAC10.* Pictures were taken 72 h and 6 days after transfection with siRNAs. In negative control siRNA (NC) transfected cells, EGFP-LC3 was diffusely distributed, whereas knockdown of *HDAC10* caused a punctuate pattern indicative of redistribution of EGFP-LC3 to autophagosomes at early (72 h) as well as late (6 d) time points. (C) LC3 conversion. Western blot showing the conversion ofLC3-I to autophagosomal conjugated LC3-II 6 d after transfection with siRNAs targeting *HDAC10* (siRNA #1 and #2). NC #1: negative control siRNA #1 transfected cells. Actin was used as a loading control. Numbers indicate the ratio of LC3-II protein levels to β-Actin protein levels. (D) LAMP2A accumuation. Western Blot demonstrating increased levels of the lysosomal marker protein LAMP2A 6 days after transfection with siRNAs targeting *HDAC10* (siRNA #1 and #2). NC #1: negative control siRNA #1 transfected cells. Actin was used as a loading control. Numbers indicate the ratio of LAMP2A protein levels to β-Actin protein levels. (E) Electron microscopy. Representative pictures of BE(2)-C cells transfected with negative control siRNA #1 (NC; upper panel) or siRNA #1 against *HDAC10* (lower panel). Panels on the left side display pictures taken at lower magnification, whereas sections with higher magnification are shown on the right side. Circles in the lower magnification pictures mark the regions, which are displayed at higher magnification. Cells transfected with control siRNA showed some single autophagosomes with myelin-like inclusions. In cells with siRNA #1 against *HDAC10,* a clear increase in autophagolysosomes could already be seen at low magnification (left panel). Nuclear morphology did not change. At higher magnification (right panel), autophagosomes (AP) and autophagolysosomes (AL) with electron-dense material could be documented. Other organelles appeared unaltered at this time point (72 h post transfection).

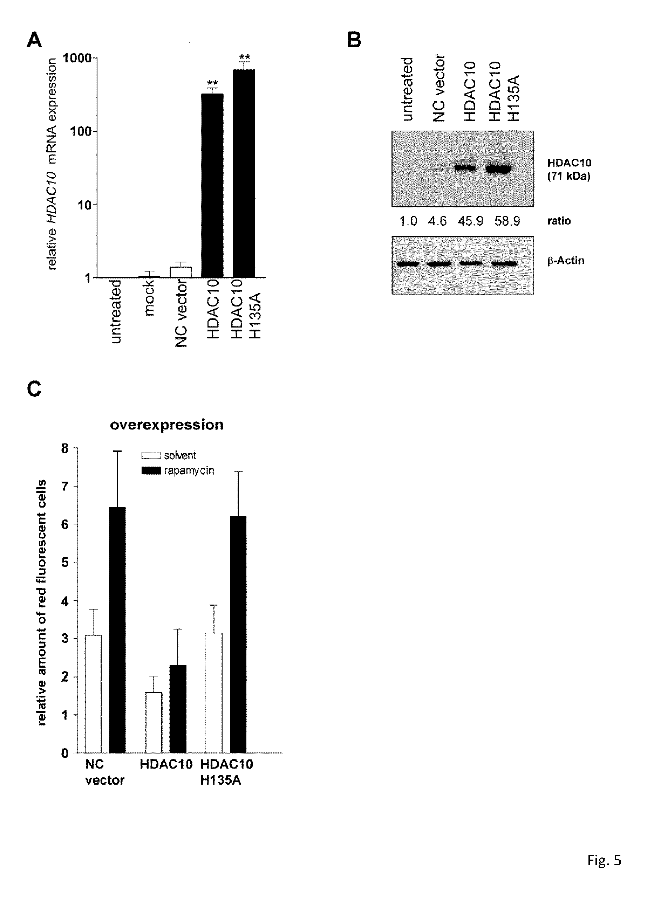

**Figure 5****: HDAC10 expression in neuroblastoma tumors and cell lines**
   (A) *HDAC10* overexpression 48 h after transfection. Transient transfection with *HDAC10* expressing plasmids (black bars) led to *HDAC10* overexpression in BE(2)-C cells. Untreated = BE(2)-C cells cultured in medium only (open bar), mock = cells treated with transfection reagent only (open bar), NC vector = cells transiently transfected with a negative control vector (open bar), HDAC10 = cells transiently transfected with *HDAC10* expressing plasmids (black bar). HDAC10 H135A = a catalytically inactive *HDAC10* variant. (B) Western Blot showing up-regulated HDAC10 protein expression upon transfection with HDAC10 plasmids. β-Actin protein levels served as loading controls. Numbers indicate the ratio of HDAC10 protein levels to β-Actin protein levels. (C) Enzymatic activity of HDAC10 contributes to the regulation of lysosomal processes in neuroblastoma cells. Forced *HDAC10* overexpression reduced endogenous levels and inhibited rapamycin (100 nM) induced accumulation of acidic lysosomes in BE(2)-C cells. A catalytically inactive *HDAC10* variant (HDAC10 H135A) did not alter the shift from green to red fluorescence. Acridine orange red fluorescent cells are represented in relation to non-transfected, solvent treated cells. Solvent (open bars) = cells treated with DMSO, rapamycin (black bars) = cells treated with 100 nM rapamycin, NC vector = cells transiently transfected with a negative control vector, HDAC10 = cells transiently transfected with *HDAC10* expressing plasmid, HDAC10 H135A = cells transiently transfected with a plasmid expressing a *HDAC10* H135A mutated version of the deacetylase domain. Error bars represent S.E.M.

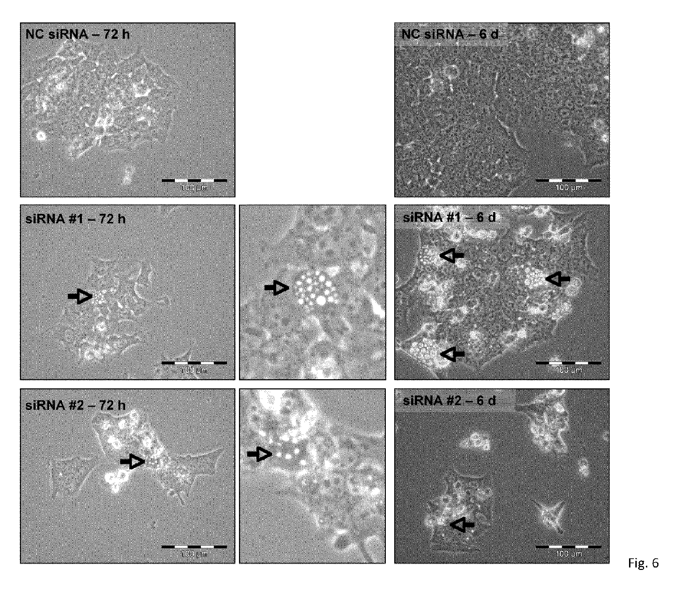

**Figure 6****: Targeting of HDAC10 induces vacuolization of BE(2)-C cells**
   Cell morphology. Knockdown of *HDAC10* expression induces morphological aberrations, such as massive cytosolic vacuolization of the cells. These aberrations were detectable 72 h after transfection with siRNAs targeting *HDAC10* (siRNA #1 and #2) and were still detectable 6 d after transfection. Cells transfected with a negative control (NC) siRNA did not display these morphological changes upon transfection. Arrows indicate cytosolic aberrations.

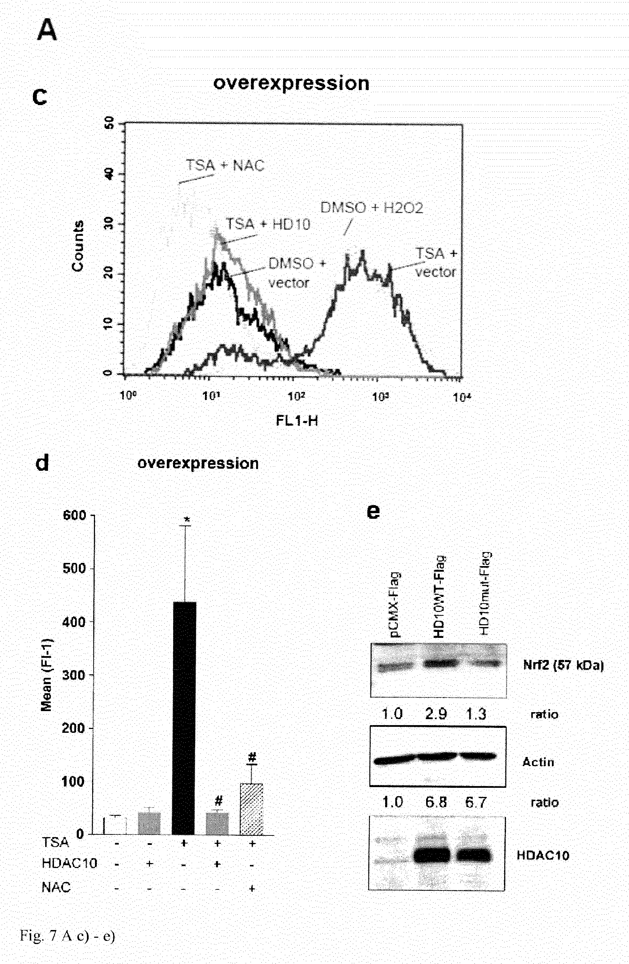

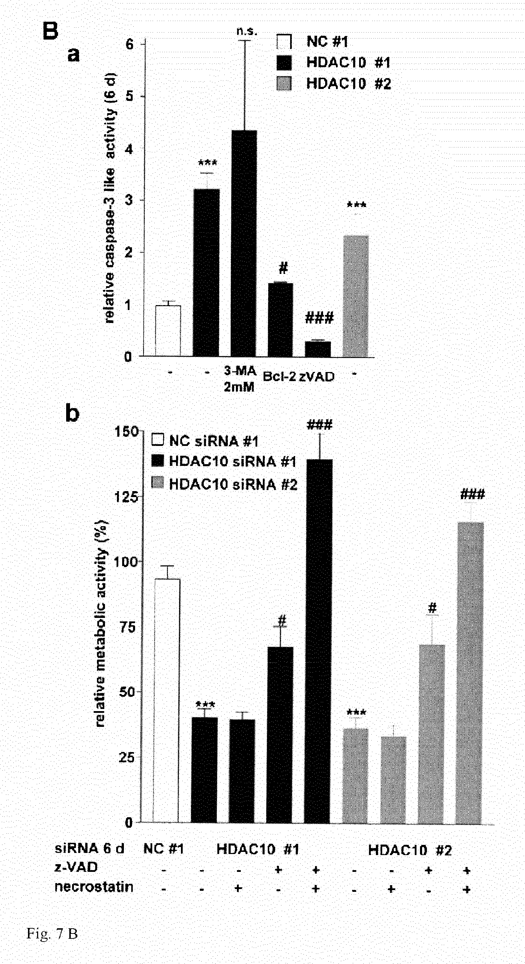

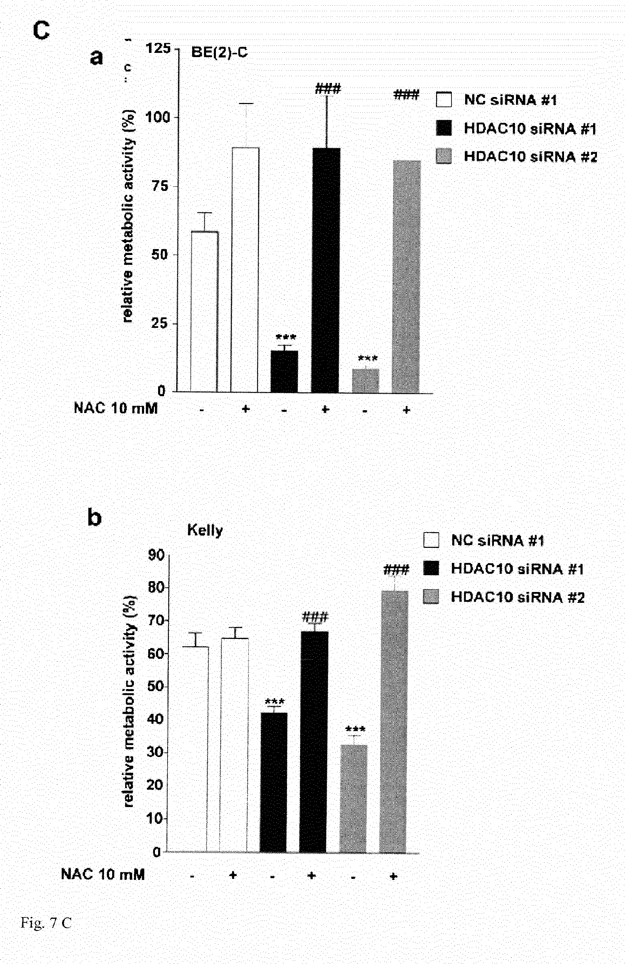

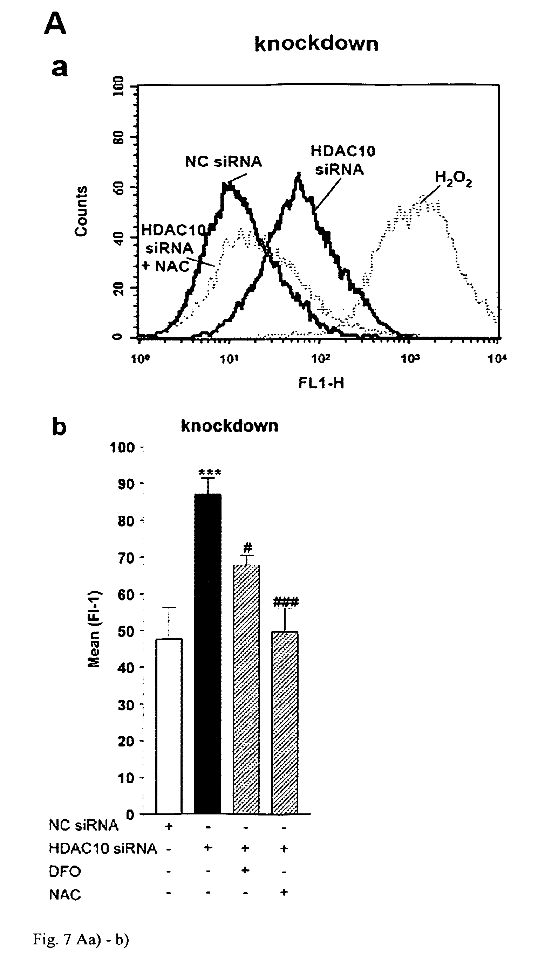

**Figure 7****: Targeting of HDAC10 induces late time-point cell death of BE(2)-C cells via lysosomal mediated ROS release**
   (A) HDAC10 knockdown causes ROS release as detected via flow cytometry. (a) Histogram reflecting the different ROS levels in BE(2)-C cells upon treatment. 72 hours after transfection with NC siRNA or *HDAC10* siRNA, cells were incubated with the oxidant-sensitive probe CM-H2DCFDA. H₂O₂ served as a positive control. Fluorescence was detected in F1-1 channel. (b) Bar diagram presenting the mean of all ROS measurements upon siRNA transfection. The antioxidants NAC and DFO served as negative controls. (c) Histogram reflecting the different ROS levels in BE(2)-C cells upon treatment. 72 hours after transient transfection with NC vector or *HDAC10*-coding plasmid (HD10) and 24 hours after TSA (75 nM) treatment, cells were incubated with the oxidant-sensitive probe CM-H2DCFDA. H₂O₂ served as a positive control. Fluorescence was detected in F1-1 channel. (d) Bar diagram presenting the mean of all ROS measurements upon *HDAC10* overexpression and TSA co-treatment. The antioxidant NAC served as a negative control. (e) Nrf2 detection. Western Blot showing increased levels of Nrf2 48 h after transfection with NC vector (pCMX-Flag) or HDAC10-coding plasmids (WT = wildtype, mut = catalytic site mutation H135A). (B) HDAC10 knockdown induces death of BE(2)C cells (a) DEVD-ase activity of HDAC10 siRNA transfected cells was measured 6 days after transfection with siRNAs and 96 h after ectopic Bcl-2 expression or 48 h after co-treatment with caspase inhibitor z-VAD (20 µM), respectively. 3-MA (2 mM) was used to inhibit macroautophagy. 3-MA was added on day 2 and day 4 after transfection. Caspase activity is represented in relation to the basal activity of untreated cells. Asterisks: unpaired two-tailed t-test *HDAC10* siRNA versus NC siRNA, * P<0.05; ** P < 0.01; *** P < 0.001. Number sign: unpaired two-tailed t-test *HDAC10* siRNA versus *HDAC10* siRNA with co-treatment, # P < 0.05; ### P < 0.001. Error bars represent S.E.M. (b) Metabolic activity (WST-1 assay) 6 d post-transfection. Cells were transfected with siRNA #1 (black bar) and #2 (grey bar) targeting *HDAC10.* On day 4 and 5 after transfection, cells were subjected to z-VAD (20 µM) in the absence and presence of necrostatin (50 µM). Metabolic activity is displayed as % activity of untreated cells. Asterisks: unpaired two-tailed t-test *HDAC10* siRNA versus NC siRNA (open bar), *** P < 0.001. Number sign: unpaired two-tailed t-test *HDAC10* siRNA versus *HDAC10* siRNA with co-treatment, # P < 0.05; ### P < 0.001. Error bars represent S.E.M. (C) The ROS scavenger NAC rescues cell death induced by HDAC10 knockdown. Metabolic activity (WST-1 assay) 6 d post-transfection of (a) BE(2)-C cells and (b) Kelly cells. The antioxidant NAC rescued from *HDAC10* knockdown (siRNA #1 = black bar, siRNA #2 = grey bar) mediated cell death. Asterisks: unpaired two-tailed t-test *HDAC10* siRNA versus NC siRNA (open bar), *** P < 0.001. Number sign: unpaired two-tailed t-test *HDAC10* siRNA versus *HDAC10* siRNA with co-treatment, # P < 0.05; ### P < 0.001. Error bars represent S.E.M.

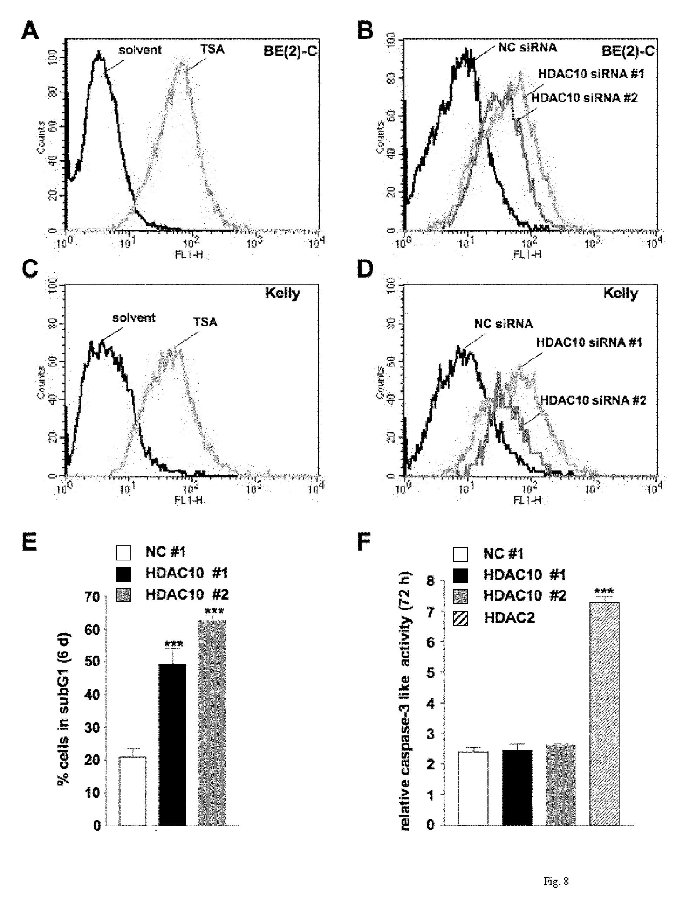

**Figure 8****: ROS and apoptosis detection in NB cell lines**
   Detection of ROS release with the oxidant-sensitive probe CM-H2DCFDA via flow cytometry. Histograms reflecting the different ROS levels in BE(2)-C (A, B) and Kelly (C, D) cells upon HDAC inhibition with TSA (24 h, 75 nM; A, C) or HDAC10 knockdown (72 h; B, D), respectively. Fluorescence was detected in F1-1 channel. (C) Nicoletti staining for cell death detection 6 d after transfection. Cells were stained with propidium iodide and the percentage of cells in the sub-G1 area of the cell cycle profile is displayed. Open bar = negative control (NC #1) siRNA transfected BE(2)-C cells, black bar = cells transfected with *HDAC10* siRNA #1, grey bar = cells transfected with HDAC10 siRNA #2. Asterisks: unpaired two-tailed t-test *HDAC10* siRNA versus NC siRNA, *** P < 0.001. Error bars represent S.E.M. (D) No changes in caspase activity 72 h post transfection. *HDAC10* siRNA #1 (black bar) and #2 (grey bar) transfected BE(2)-C cells displayed no change in DEVD-ase activity compared with the negative control siRNA (NC #1, open bar). The positive apoptosis control refers to *HDAC2* knockdown (siRNA *HDAC2,* hatched bar). Caspase activity is represented in relation to the activity of untreated cells. Asterisks: unpaired two-tailed t-test HDAC siRNA versus NC siRNA; ** P < 0.01. Error bars represent S.E.M.

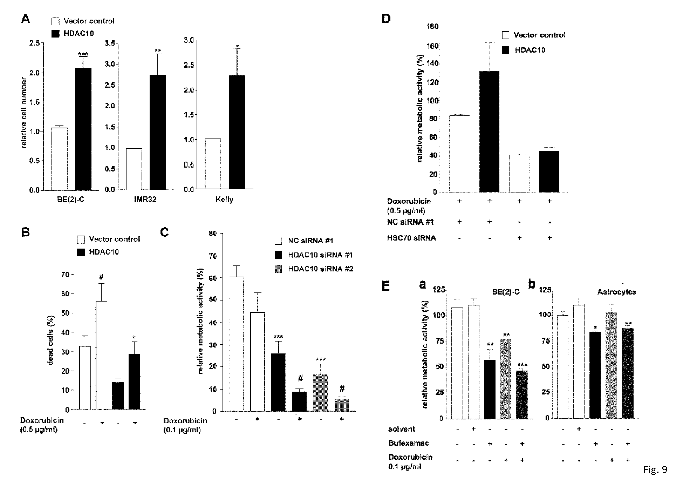

**Figure 9****: Targeting of HDAC10 sensitizes NB tumor cells to cytotoxic drug tretament**
   (A) HDAC10 expression increases survival of NB cells. Transfection of BE(2)-C (left panel), IMR32 (middle panel) and Kelly (right panel) cells with *HDAC10*-coding plasmid (black bar) increased cell population growth of both neuroblastoma cell lines compared to negative control vector (NC vector, open bar) transfected cells. Population growth is presented as cell numbers of transfected cells in relation to cell numbers of control cells. Asterisks: unpaired two-tailed t-test TSA versus solvent, *** P < 0.001. Error bars represent S.E.M. (B) HDAC10 protects NB cells against cytotoxic drug treatment. BE(2)-C were transfected with negative control vector (NC vector, open bar) or *HDAC10* expressing plasmid (black bar). Trypan blue positive and negative cells were counted 72 h after transfection and 48 h after doxorubicin (0.5 µg / ml) treatment. The percentage of viable cells in relation to total cell numbers is presented. Asterisks: unpaired two-tailed t-test vector control plus doxorubicin versus *HDAC10* plus doxorubicin, * P < 0.05. Number sign: unpaired two-tailed t-test solvent versus doxorubicin, # P < 0.05. Error bars represent S.E.M. (C) Targeting of HDAC10 sensitizes NB cells for cytotoxic drug treatment. Cells were transfected with siRNAs targeting *HDAC10* (siRNA #1 = black bar, siRNA #2 = grey bar). On day 3 after transfection, cells were treated with doxorubicin (0.1 µg / ml). Metabolic activity (WST-1 assay) is displayed as % activity of untreated cells. Asterisks: unpaired two-tailed t-test *HDAC10* siRNA versus NC siRNA, *** P < 0.001. Number sign: unpaired two-tailed t-test *HDAC10* siRNA versus *HDAC10* siRNA plus doxorubicin, # P < 0.05. Error bars represent S.E.M. (D) Chaperone-mediated autophagy is involved in HDAC10 mediated survival of NB cells. BE(2)-C cells were transfected with siRNA targeting *HSC70* and co-transfected with negative control vector (NC vector, open bar) or *HDAC10* expressing plasmid (black bar). On day 4 after transfection, cells were treated with doxorubicin (0.5 µg / ml). Metabolic activity (WST-1) was measured 6 days after transfection and is displayed as % activity of untreated cells. (E) Tumor cell selective sensitization to doxorubicin via targeting of class IIb HDACs. Metabolic activity (WST-1 assay) after 3 days of bufexamac (class IIb HDAC6/10 inhibitor, 100 µM) treatment of (a) BE(2)-C or (b) astrocytes, respectively. Cells were co-treated with doxorubicin (0.1 µg / ml) 24 h before activity assay. Metabolic activity is displayed as % activity of untreated cells. Asterisks: unpaired two-tailed t-test drug treatment versus solvent control, * P < 0.05; ** P < 0.01; *** P < 0.001. Error bars represent S.E.M.

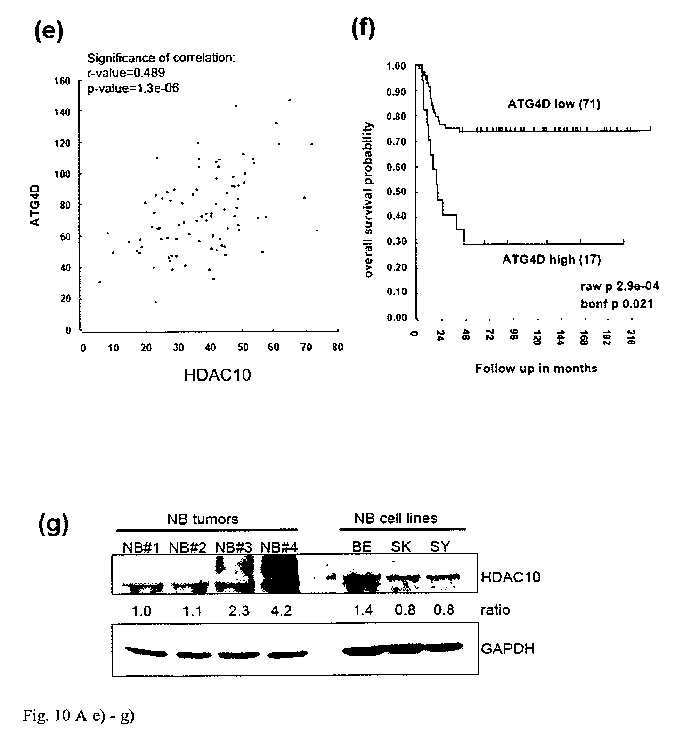

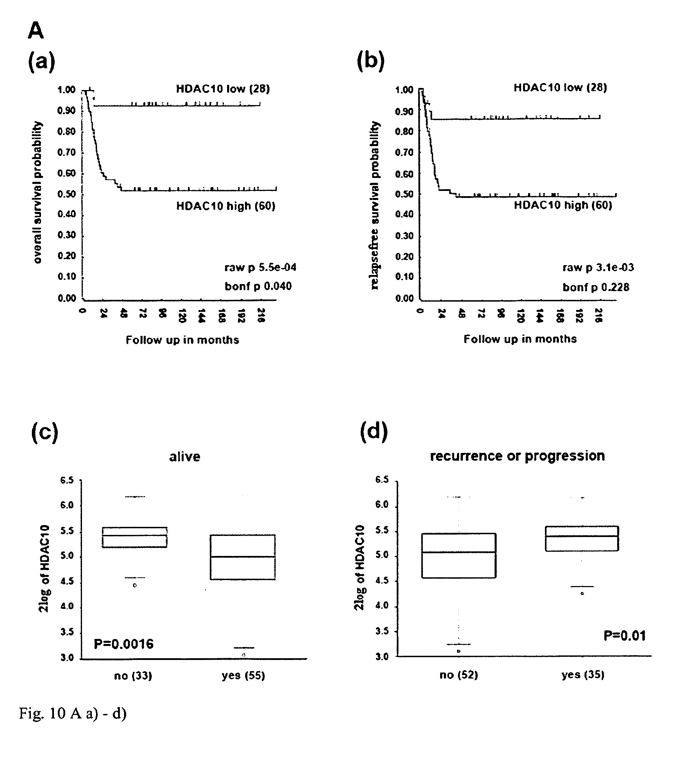

**Figure 10****: In vivo relevance of HDAC10 expression in NB patient samples**
   (A) (a) - (f) Expression data from web-based R2 microarray analysis and visualization platform (http://r2.amc.nl). Kaplan-Meier curves of (a) overall and (b) relapse-free patient survival for low versus high *HDAC10* expression in 88 NB patients. Scan modus for cut-off determination with bonferroni correction of p-values for multiple testing was used. The raw p-value gives the logrank statistical significance of obtaining a Kaplan curve as drawn. The bonferoni corrected p-value, denotes the significance in survival potential when performing a scan. The association of high *HDAC10* expression with poor prognosis is statistically significant. (c) Box-plot showing a significant difference in HDAC10 expression in tumors of patients which are alive (yes) and which are not (no). (d) Box-plot showing a significantly higher expression of *HDAC10* in tumors of patients with recurrence or progression. (e) Kaplan-Meier curves of overall patient survival for low versus high *ATG4D* expression. Scan modus for cut-off determination with bonferroni correction of p-values for multiple testing was used. The association of high *ATG4D* expression with poor prognosis is statistically significant. (f) Graph showing a significant correlation of *HDAC10* with *ATG4D* expression. (g) Western Blot. Neuroblastoma (NB) tumor samples as well as NB cell lines expressed detectable levels of HDAC10. GAPDH protein levels served as loading controls. NB#1-4 = Tumor samples from NB patients of the German NB trial. BE = BE(2)-C, SK = SK-N-BE(2), SY = SH-SY5Y. Numbers indicate the ratio of HDAC10 protein levels to GAPDH expression levels. (B) Scheme of HDAC10 regulated CMA and its consequences for NB cell death. Targeting of HDAC10 interferes with lysosomal Hsc70 acetylation as well as intralysosomal localization and induces lysosomal dysfunctions leading to the inhibition of autophagic flux, accumulation of autophagosomes, ROS release and NB cell death. Furthermore, targeting of HDAC10 enhances drug induced tumor cell specific toxicity via inhibition of autophagic flux and induction of lysosomal mediated cell death.
**Figure 11****:**
   (**A**) Kaplan-Meier curves of overall and (**B**) relapsefree patient survival for low versus high *HDAC10* expression in high-risk INSS stage 4 neuroblastoma patients. Scan modus for cut-off determination with bonferroni correction of p-values for multiple testing was used. The association of high *HDAC10* expression with poor prognosis is statistically significant. (**C**) Kaplan-Meier curves of overall and (**D**) relapsefree patient survival for low versus high *MYCN* expression in high-risk INSS stage 4 neuroblastoma patients. Scan modus for cut-off determination with bonferroni correction of p-values for multiple testing was used. The association of high *MYCN* expression with poor prognosis is statistically not significant.

### EXAMPLES

The following Examples shall merely illustrate the invention. They shall not be construed, whatsoever, to limit the scope of the invention.

### Example 1: Material and Methods

### Cell lines and reagents

Human neuroblastoma cell lines BE(2)-C (ECACC), SH-SY5Y (DSMZ), Kelly (DSMZ) and IMR32 (DSMZ) were cultured under standard conditions (DMEM with L-glutamine and 4.5 g/l glucose containing 10% fetal bovine serum and 1% NEAA). Astrocyte culture medium additionally contained 1% astrocyte growth supplement (AGS). SK-N-BE(2) cells were obtained from the laboratory of A. Eggert, Department of Pediatric Oncology and Haematology, University Children's Hospital Essen, Germany. All neuroblastoma cell lines were verified using DNA finger-printing by the DSMZ, Germany. Human astrocytes derived from a child were obtained from J. Lacroix, Division of Tumor Virology, DKFZ, Heidelberg, Germany.

HDAC inhibitors trichostatin A (TSA), suberoylanilide hydroxamic acid (SAHA), MS-275 (Entinostat) and valproic acid (VPA) were used as previously described (Deubzer, HE, Ehemann, V, Kulozik, AE, Westermann, F, Savelyeva, L, Kopp-Schneider, A et al., (2008) Anti-neuroblastoma activity of Helminthosporium carbonum (HC)-toxin is superior to that of other differentiating compounds in vitro. Cancer Letters). Selective HDAC8 inhibitor (Compound 2) is described elsewhere (Krennhrubec, K, Marshall, BL, Hedglin, M, Verdin, E and Ulrich, SM, (2007) Design and evaluation of 'Linkerless' hydroxamic acids as selective HDAC8 inhibitors. Bioorg Med Chem Lett 17: 2874-8). Tubacin was obtained from A. Schwienhorst and C. Hildmann, Institute of Molecular Genetics and Preparative Molecular Biology, University of Göttingen, Germany. The compound was dissolved in DMSO as a stock of 10 mM. PCI-24781 (10 mM) was obtained from Pharmacyclics (USA) and were dissolved in DMSO. Panobinostat (1 mM, Selleck), bufexamac (1 M, Sigma), rapamycin (100 µM, Sigma), epoxomicin (1 mM, Calbiochem), z-VAD (100 mM, MBL), necrostatin (50 mM, Alexis), CA-Inhibitor (20 mM), and Bafilomycin A1 (10 µM, Sigma Aldrich) were dissolved in DMSO. Chloroquine (100 mM, Sigma), NAC (1 M, Sigma), and Doxorubicin (1 mg/ml, Biozol) were dissolved in H₂O.

### Transient Transfections

Transfection with small interfering RNA (siRNA). 6x10⁴ cells/well were seeded into 6-well plates 24 h before transient transfection with 25 nM siRNA for 18 h using HiPerFECT transfection reagent according to the manufacturer's instructions (Qiagen, Hilden, Germany). In 96-well plates the reverse transfection protocol as indicated in the manufacturer's instructions, was used. For *HDAC10* knockdown, the following siRNAs were used: siRNA #1 (ID 33581, Exon14/15, Ambion (Europe) Ltd., Huntingdon, Cambridge, UK); siRNA #2 (ID 120681, Exon5, Ambion). For knockdown of all other classical HDACs the following siRNAs were used: *HDAC1* Hs_HDAC1_6 (Qiagen), *HDAC2* #120209 (Ambion)(Oehme, I, Deubzer, HE, Wegener, D, Pickert, D, Linke, JP, Hero, B et al., (2009) Histone deacetylase 8 in neuroblastoma tumorigenesis. Clin Cancer Res 15: 91-9), *HDAC3* #120349 (Ambion), *HDAC4* #107926 (Ambion), *HDAC5* Hs_HDAC5_1 (Qiagen)(Milde, T, Oehme, I, Korshunov, A, Kopp-Schneider, A, Remke, M, Northcott, P et al., (2010) HDAC5 and HDAC9 in medulloblastoma: novel markers for risk stratification and role in tumor cell growth. Clin Cancer Res 16: 3240-52), *HDAC6* #120451 (Ambion), *HDAC7* Hs_HDAC7A_6 (Qiagen), *HDAC8* #120599 (Ambion)(Oehme et al. (2009), op. cit.), *HDAC9* Hs_HDAC9_1 (Qiagen)(Milde et al.; cit. loc.), *HDAC11* Hs_HDAC11-6 (Qiagen). For the targeting of *HSC70 (HSPA8)* the siRNA with the sequence: sense 5'-GAU CGA UUC UCU CUA UGA ATT-3' (SEQ ID NO: 8), antisense 5'-UUC AUA GAG AGA AUC GAU CTT-3' (SEQ ID NO: 9; Ambion) and for *LAMP2A* with the sequence sense 5'-CCA UCA GAA UUC CAU UGA ATT-3' (SEQ ID NO: 10), antisense 5'-UUC AAU GGA AUU CUG AUG GTT-3' (SEQ ID NO: 11;Ambion) was applied. As negative controls the following siRNAs were used: RF (siCONTROL RISC-Free siRNA #1, Dharmacon); NC #1 (Silencer® Negative Control #1, Ambion); NC #5 (Silencer® Negative Control #5, Ambion). Transfection efficacy in BE(2)-C cells was 90,3 +/- 2,3 % as determined by fluorescently labeled siRNA siGLO Lamin A/C (Dharmacon).

Transfection with cDNA containing plasmids. 2x10⁵ cells/well were seeded into 6-well plates 24 h before transient transfection with plasmid-DNA using Effectene (Qiagen). For *HDAC10* overexpression, the C-terminal FLAG-tagged HDAC10 pCMX construct and the C-terminal FLAG-tagged HDAC10 H135A mutant construct (Guardiola, AR and Yao, TP, (2002) Molecular cloning and characterization of a novel histone deacetylase HDAC10. J Biol Chem 277: 3350-6) provided by T.-P. Yao, Department of Pharmacology and Cancer Biology, Duke University Medical Center, North Carolina, were used. As a negative control vector, pCMX-FLAG (provided by R. Schuele, University of Freiburg Medical Center, Germany) was applied. For EGFP-LC3 overexpression, the EGFP-LC3 construct (Jackson, WT, Giddings, TH, Jr., Taylor, MP, Mulinyawe, S, Rabinovitch, M, Kopito, RR et al., (2005) Subversion of cellular autophagosomal machinery by RNA viruses. PLoS Biol 3: e156) from Addgene (Cambridge, USA) was used. *BCL2* overexpression was achieved by using the pcDNA-BCL2 construct obtained from the DKFZ Vector and Clone Repository.

### Real time reverse transcription-polymerase chain reaction (RT-PCR) analysis of mRNA

Real Time RT-PCR was performed as described previously (Oehme et al.(2009), op. cit.). The following specific primer pairs were used: *HDAC10* (forward: 5'-ATC TCT TTG AGG ATG ACC CCA G-3' (SEQ ID NO: 12), reverse: 5'-ACT GCG TCT GCA TCT GAC TCTC-3' (SEQ ID NO: 13)), *HDAC6* (forward: 5'-CAA GGA ACA CAG TTC ACC TTC G-3' (SEQ ID NO:14), reverse: 5'-GTT CCA AGG CAC ATT GAT GGT A-3' (SEQ ID NO: 15), *HSC70 (HSPA8)* (forward: 5'- TGC TGC TGC TAT TGC TTA CG-3'(SEQ ID NO: 16); reverse: 5'-TCA ATA GTG AGG ATT GAC ACA TCA-3' (SEQ ID NO:17), *LAMP2A* (QT00077063, Qiagen), *SDHA* (succinate dehydrogenase complex, subunit A, forward: 5'-TGG GAA CAA GAG GGC ATC TG-3'(SEQ ID NO: 18), reverse: 5'-CCA CCA CTG CAT CAA ATT CAT G-3' (SEQ ID NO: 19)) and *HPRT* (hypoxanthine phosphoribosyltransferase 1, forward: 5'-TGA CAC TGG CAA AAC AAT GCA-3' (SEQ ID NO: 20), reverse: 5'-GGT CCT TTT CAC CAG CAA GCT-3'(SEQ ID NO 21)). Data were analyzed using Applied Biosystems Prism software and the ΔΔC_{T} method. Target gene expression was normalized against *SDHA* and *HPRT,* which are consistently expressed in primary neuroblastoma (Fischer, M, Skowron, M and Berthold, F, (2005) Reliable transcript quantification by real-time reverse transcriptase-polymerase chain reaction in primary neuroblastoma using normalization to averaged expression levels of the control genes HPRT1 and SDHA. J Mol Diagn 7: 89-96). The difference between mean threshold PCR cycle values for target and control genes gave the ΔC_{T} value.

### Immunoprecipitation and Immunoblotting

Immunoprecipitation. Cells were lysed in buffer containing 50 mM Tris-HCl (pH 7.4), 150 mM NaCl, 1 mM EDTA, 1% Triton X-100, supplemented with protease inhibitor cocktail. Total cell lysates were pre-cleared for 1 h with anti-rabbit IgG TrueBlot beads (eBioscience) and then incubated for 2.5 h with antibodies at 4°C and complexes were captured with anti-rabbit IgG TrueBlot beads (eBioscience) or alternatively with anti-FLAG M2 conjugated agarose (Sigma) by incubation at 4°C for 2 h.

Western blot analysis was performed as described previously (Oehme et al. (2009), op. cit.). The following antibodies were used: anti-HDAC10 (Sigma), anti-LC3 (Sigma), anti-HDAC6 (Cell Signaling), anti-p62/SQSTM1 (MLB), anti-Hsc70 (Santa Cruz), anti-FLAG-M2 (Sigma) anti-GAPDH (Millipore), anti-Ac-Lysine (rabbit, Cell Signaling) and anti-β-Actin (clone AC-15, Sigma). β-Actin expression served as loading control. Ratios were calculated with the Bio-1D (Version 12.10a) Software. Protein extracts from primary neuroblastoma tumors from patients with the following characteristics were used: NB#1: age 63 days, stage 1, MYCN single copy. NB#2: age 49 days, stage 1, MYCN single copy. NB#3: age 13 years, stage 4, MYCN single copy. NB#4: age 5 years, stage 4, MYCN single copy.

### Cell counting, cell viability and cell death assays

Growth curves and colony formation. Growth curve and colony formation analysis of BE(2)-C cells was performed as described previously (Deubzer, HE, Ehemann, V, Westermann, F, Heinrich, R, Mechtersheimer, G, Kulozik, AE et al., (2008) Histone deacetylase inhibitor Helminthosporium carbonum (HC)-toxin suppresses the malignant phenotype of neuroblastoma cells. Int J Cancer 122: 1891-900,30, Oehme et al.(2009), op. cit.).

Viabilty assay. Trypsinized cells were pooled with corresponding supernatant, centrifuged and resuspended in 1.5 ml cell culture media. Cell viability was measured by automated trypan blue staining with Vi-Cell XR Cell Viability Analyzer from Beckman Coulter (Krefeld, Germany).

WST-1 Asssay. Metabolic activity was determined using the colorimetric WST-1 assay kit from Roche (Mannheim, Germany) as described in the manufacturer's manual.

Nicoletti staining. Apoptosis quantification by flow cytometry was performed as described previously (Oehme, I, Bosser, S and Zornig, M, (2006) Agonists of an ecdysone-inducible mammalian expression system inhibit Fas Ligand- and TRAIL-induced apoptosis in the human colon carcinoma cell line RKO. Cell Death Differ 13: 189-201).

Caspase-3-like protease activity assay. Caspase-3-like protease activity was measured with the Caspase-3 Fluorometric Assay (BioVision) according to the manufacturer's protocol modified as described previously (Oehme et al.. (2006), op. cit.). For cell death detection adherent as well as floating cells were collected.

### Acridine orange, GFP-LC3and ROS detection

For quantification of acidic lysosomes, the amount of green respectively red fluorescence was determined, since the acidification of (auto-)lysosomes causes a shift from green to red fluorescence. Acridine orange accumulates in lysosomal particles and the quenching of its fluorescence correlates with the DpH across the lysosomal membrane (Moriyama, Y, Takano, T and Ohkuma, S, (1982) Acridine orange as a fluorescent probe for lysosomal proton pump. J Biochem 92: 1333-6). Therefore, the cytoplasm and nucleolus glow bright green, whereas acidic compartments shine bright red. By comparing the mean red-to-green fluorescence ratio within different cell populations, detection and quantification of the acidic compartment is possible. Cells were stained with phenol red-free medium containing 1 µg/ml acridine orange (Sigma) at 37°C for 30 min, collected and resuspended in 300 µl phenolred-free medium for FACS analysis. The green (510-530 nm) and the red (>650 nm) fluorescent emission of living cells, stimulated with blue laser (488 nm), were measured with FACSCalibur (Becton Dickinson, San José, CA) and analyzed with CellQuest Pro.

GFP-LC3. GFP-fluorescence of living cells was measured with FACS Calibur (Fl-1: 510-530 nm) and analyzed with CellQuest Pro.

ROS generation was quantified using CM-H₂DCFDA (Molecular Probes) according to the manufacturer's instructions. Cells were incubated for 20 min with 5 µM dye and fluorescence intensity was measured with FACSCalibur and analyzed with CellQuest Pro.

### Electron and Fluorescent microscopy

Electron microscopy. 6x10⁴ cells/well were seeded on glass slides in 6-well plates. 72 h after transfection, cells were fixed in Karnovsky's solution (4% PFA, 2% glutaraldehyde), postfixed in 2% osmium tetroxide in cacodylate buffer (0.2 M), dehydrated in graded ethanols and embedded in araldite. Ultrathin sections (60-70 nm) were cut on an ultracut UCT (Leica). Sections were contrasted with uranyl acetate and lead citrate, and analyzed with an EM 900 Zeiss electron microscope.

Fluorescent microscopy. EGFP-LC3 transfected cells were analyzed with the Olympus CKX41 microscope, 40x objective lense and ColorView I FW camera. CellB* imaging software was used for the acquisition of microscopic images.

Immunostaining and colocalization studies. After fixation with 2% para-formaldehyde in PBS and permeabilization with 0.1% Triton X-100 in PBS, cells were incubated with primary antibodies for 2 h at room temperature and then with the appropriate secondary antibody for 1 h at room temperature. Cells were examined using a laser scanning confocal microscope (Zeiss LSM700, 63x objective lense). Primary antibodies: anti-Hsc70 (rabbit, Santa Cruz) and anti-LAMP2 (mouse, Santa Cruz). Secondary antibodies: Cy3-labeled anti-rabbit antibody and Alexa488 anti-mouse antibody.

### In vitro HDAC10 activity assay

The inhibitory activity of compounds toward HDAC10 was investigated by Reaction Biology Corporation (Malvern, PA, USA).

### Statistical analysis

### All cell culture experiments were performed in duplicates or triplicates and each experiment was repeated at least thrice. For statistical analysis, a two-tailed t-test of significance was performed to compare two treatments, using GraphPad Prism version 3.0a. P-values less than 0.05 were considered as statistically significant (***P ≤ 0.001; **0.001 ≤ P ≤ 0.01; *0.01 ≤ P ≤ 0.05). Means are represented in bar charts and error bars represent the standard error of the mean (SEM).

### Example 2: Global HDAC inhibition leads to accumulation of acidic lysosomes in neuroblastoma cell lines

It has been shown that broad spectrum HDAC inhibitors (HDACi) induce neuroblastoma (NB) cell death (Deubzer et al., op. cit., Subramanian, C, Jarzembowski, JA, Opipari, AW, Jr., Castle, VP and Kwok, RP, (2007) CREB-binding protein is a mediator of neuroblastoma cell death induced by the histone deacetylase inhibitor trichostatin A. Neoplasia 9: 495-503). As pan-HDACi have been reported to induce signs of autophagy in cells of different tumor entities, we investigated the autophagic response in NB cells upon TSA treatment. Indeed, TSA (75 nM, 24 h) induced the accumulation of acridine orange red-fluorescent acidic lysosomes in all NB cell lines tested (Fig. 1A, black bars) to a similar extend compared with the positive controls rapamycin and chloroquine (Fig. 2) and was completely inhibited by lysosomal proton pump inhibitor Bafilomycin A1, pointing towards a lysosomal-mediated shift to red-fluorescence (Fig. 2).

In order to narrow down the relevant HDACs involved, BE(2)-C were treated with a panel of inhibitors targeting different HDAC family members. Notably, only the pan-HDACi TSA, PCI-24781, panobinostat and SAHA (vorinostat), targeting a broad spectrum of HDACs, including class IIb HDAC10 (Table 1), induced accumulation of acidic lysosomes in BE(2)-C cells (Fig. 1B). In contrast, inhibitors excluding HDAC10 from their inhibitory profile (MS-275 (entinostat), Compound2, PCI-34051, VPA and tubacin), did not (Table 1 and Fig. 1B). In line with these findings, bufexamac, a recently identified class IIb inhibitor (Bantscheff, M, Hopf, C, Savitski, MM, Dittmann, A, Grandi, P, Michon, AM et al., (2011) Chemoproteomics profiling of HDAC inhibitors reveals selective targeting of HDAC complexes. Nat Biotechnol 29: 255-65), targeting HDAC6 and HDAC10, also induced a significant increase in acidic lysosomes (Table 1 and Fig. 1B). This result indicates that only the treatment with HDACi, covering HDAC10 in their inhibitory profile, induced the accumulation of acidic lysosomes. To confirm the specific role of HDAC10, knockdown experiments with siRNAs targeting all classical HDACs 1-11 were performed (Fig. 1C). The knockdown of *HDAC10* induced the highest amount of red fluorescent cells, corresponding to acidic lysosomes (Fig. 1C). In addition, minor changes were observed with HDAC1 and 3 siRNA.

As the regulation of lysosomal activities of tumor cells can differ from normal cells, human astrocytes were treated next with HDAC class IIb-specific inhibitor bufexamac. Indeed, astrocytes responded only with a very moderate shift to red fluorescence compared with neuroblastoma cells, suggesting a tumor specific function of class IIb HDACs on regulation of lysosomal activity (Fig. 1D).

In summary, targeting of HDAC10 either through small molecule inhibitors or through siRNAi-mediated knockdown induced the accumulation of acidic lysosomes in NB cell lines, but not in untransformed astrocytes.

### Example 3: HDAC10 regulates the formation of acidic lysosomes

To further investigate the functional role of HDAC10 in lysosome formation, BE(2)-C cells were transiently transfected with two siRNAs targeting different regions of the *HDAC10* mRNA sequence. The specific knockdown of *HDAC10* expression (Fig. 3A) did not influence the mRNA levels of the other class IIb family member *HDAC6,* which has the highest sequence homology to *HDAC10* (Fig. 3B). Efficient reduction of HDAC10 protein levels was detectable 48 h post transfection (Fig. 3C) and persisted at least for 6 days (Fig. 3D). The siRNA-mediated *HDAC10* knockdown induced the accumulation of acidic lysosomes 72 h after transfection (Fig.. 4A and Fig. 3E). The acidification was completely blocked by the lysosomal proton pump inhibitor Bafilomycin A1 (Fig.. 4A). Six days after transfection, living BE(2)-C cells still displayed an increased amount of acidic lysosomes (Fig.. 4A). These results were additionally confirmed in Kelly NB cells (Fig. 3F). Vice versa, ectopic expression of wild-type HDAC10 reduced endogenous autolysosome formation, whereas a mutated variant of the catalytic domain (HDAC10 H135A) did not (Fig.. 5). In addition, HDAC10 overexpression completely abolished rapamycin induced autolysosme formation, whereas the catalytically mutant construct did not (black bars; Fig.. 5). This indicates that the catalytic domain of HDAC10 is required for the regulation of autolysosome formation.

To investigate whether the accumulation of acidic lysosomes upon knockdown of *HDAC10* expression is part of macroautophagic processes, BE(2)-C cells were analyzed for markers of macroautophagy. During the formation of autophagosomes, LC3 is lipidated and the LC3- phosphatidylethanolamine conjugate (LC3-II) is localized to the membrane of autophagosomes. Transfection of BE(2)-C cells with siRNA targeting *HDAC10* induced the recruitment of a EGFP-LC3 fusion protein to autophagic organelles, indicated by the cytoplasmatic, punctated pattern of EGFP-LC3 (Fig.. 4B). The accumulation of LC3-positive autophagosomes increased over time (Fig.. 4B 6d post transfection). The increase of the faster SDS-PAGE migrating form LC3-II was detected by immunoblotting (Fig.. 4C). The accumulation of cytoplasmic vacuolization of the cells, another characteristic feature of macroautophagy, also increased over time (Fig.. 6). The accumulation of lysosomes was confirmed via detection of increased LAMP2 expression, a lysosomal specific protein (Fig.. 4D). To proof the formation of autophagosomes as well as acidic lysosomes, cells were analyzed by electron microscopy. The accumulation of autophagosomes and a massive increase in electron-dense lysosomes was indeed detected 72 h upon *HDAC10* knockdown (Fig.. 4E). Furthermore, electron microscopy revealed intact nuclear morphology, indicative of absence of apoptosis at this 72 h time point. These data give evidence for an important regulatory function of HDAC10 in the formation of autophagic organells, such as autophagosomes and acidic (auto-)lysosomes.

### Example 4: Knockdown of HDAC10 expression leads to lysosomal-mediated ROS release and cell death in NB cells

The data so far show that HDAC10 regulates lysosomal protein degradation pathways via Hsc70 interaction in neuroblastoma cells. In addition, morphological signs of cell death under the microscope when knocking down HDAC10 were repeatedly observed. Thus, it was hypothesized that targeting of HDAC10 led to lysosomal dysfunctions leading to cell death. It was therefore investigated whether lysosomes were disrupted and intralysosomal components released. Indeed, NB cell lines significantly produced ROS already 72 hours after transfection with HDAC10 siRNA (Fig 7A (a),(b) and Fig.. 8). ROS production by HDAC10 targeting was inhibited by DFO, which specifically inhibits the generation of lysosomal ROS as well as by the antioxidant NAC (Fig.. 7A (a, b)). Of note, Cathepsin release was not detectable (data not shown), pointing towards partly but not fully disrupted lysosomal membranes.

It is known that pan-HDACi treatment induces ROS generation in tumor cells (Bolden, JE, Peart, MJ and Johnstone, RW, (2006) Anticancer activities of histone deacetylase inhibitors. Nat Rev Drug Discov 5: 769-84) (Fig.. 8). To investigate, whether the overexpression of HDAC10 is sufficient to inhibit TSA-induced ROS production, NB cells were transiently transfected with an *HDAC10* encoding plasmid or NC vector, respectively. The enforced *HDAC10* completely blocked TSA-induced ROS generation, pointing towards an essential role of HDAC10 in this process (Fig. 7A (c) and (d)).

It was next asked what the mechanism of blocked ROS-generation upon HDAC10 expression could be. In this context, Nrf2 has been described as an oncogene induced transcription factor leading to enhanced ROS detoxification in tumor cells via up-regulation of thioredoxin and glutathion. Indeed, the ectopic expression of wild-type *HDAC10,* but not of the mutated version, induced the up-regulation of Nrf2 expression (Fig.. 7e). Hence, the upregulation of Nrf2 might account for the quenching of TSA-generated ROS upon *HDAC10* overexpression in NB cells.

Because partly disrupted lysosomes are described to induce lysosomal cell death, it was next investigated the biological outcome of NB cells following targeting of HDAC10. Over a period of 6 days, the knockdown of *HDAC10* led to a significant increase in apoptotic BE(2)-C cells, measured via caspase-3 like activity (Fig.. 7Ba), propidium iodide staining (Fig.. 8E) and metabolic activity assay (Fig.. 7Bb). The ectopic overexpression of anti-apoptotic *BCL2* as well as the treatment of the cells with the global caspase inhibitor zVAD abolished *HDAC10*-knockdown mediated induction of apoptosis (Fig.. 7Ba). At earlier time points (48 h - 96 h) no caspase activation was detectable (Fig.. 8F and data not shown). Of note, as expected from results shown in Fig. 3, the inhibition of autophagy via treatment with 3-MA did not affect the induction of cell death upon targeting of HDAC10 (Fig.. 7Ba). Next, metabolic activity assay was used to investigate whether the inhibition of apoptosis upon zVAD-treatment is sufficient to abolish cell death induced via targeting of HDAC10 in NB cells. The assay revealed that the co-treatment with zVAD partly restored viability upon *HDAC10* knockdown and that the combination of zVAD and necrostatin was able to completely rescue BE(2)-C cells from *HDAC10* knockdown mediated cell death (Fig 5Bb). These results point towards an induction of apoptosis as well as necroptosis in BE(2)-C cells transfected with siRNAs targeting HDAC10 and co-treated with zVAD. In order to investigate whether ROS release, which was already detectable 72 h after transfection with HDAC10 siRNAs, is responsible for the subsequent induction of cell death 6 days after transfection in NB cells, BE(2)-C (Fig.. 7C (a)) and Kelly (Fig.. 7C (b)) cells were co-treated with the antioxidant NAC on day 4 and 5 and metabolic activity was measured on day 6 after siRNA transfection. Indeed, co-treatment with the antioxidant NAC restored viability of NB cells and abolished HDAC10 knockdown mediated toxicity. In summary, the knockdown of endogenous *HDAC10* expression decreased survival of NB cells via the induction of lysosomal ROS-mediated cell death.

### Example 5: Targeting of HDAC10 sensitizes drug-resistant NB cells for tumor cytotoxicity

The data so far show that HDAC10 regulates CMA and that the knockdown of *HDAC10* expression results in lysosomal dysregulation, inhibition of autophagic flux and induction of lysosomal cell death of NB tumor cells. Treatment of tumor cells with DNA damaging cytotoxic drugs, such as doxorubicin, induce autophagy. It has been proposed that tumor cells use autophagy as a survival mechanism to cope with drug-induced stress. As targeting of HDAC10 inhibits autophagic flux in our NB model, we hypothesized that targeting of HDAC10 would sensitize NB cells to doxorubicin induced cell death. First, it was investigated whether HDAC10 expression alone is sufficient to enhance tumor cell survival. Indeed, the enforced expression of HDAC10 significantly enhanced tumor population growth of BE(2)-C, Kelly and IMR32 cells (Fig.. 9A) and protected NB cells against doxorubicin-induced toxicity (Fig.. 9B). Vice versa, knockdown of *HDAC10* expression enhanced doxorubicin-mediated toxicity in BE(2)-C cells in a synergistic manner (Fig.. 9C). BE(2)-C cells are MYCN-amplified and MYCN-amplified cells have been described to be rather insensitive towards doxorubicin treatment (Gogolin, S, Dreidax, D, Becker, G, Ehemann, V, Schwab, M and Westermann, F, (2010) MYCN/MYC-mediated drug resistance mechanisms in neuroblastoma. Int J Clin Pharmacol Ther 48: 489-91). Moreover, BE(2)-C cells are even more resistant to doxorubicin treatment than other MYCN-amplified NB cell lines (IMR32, Kelly, Suppl. Fig.. 9).

To confirm that the cell death protective function of HDAC10 depends on functional CMA, cells were co-transfected with siRNA targeting *HSC70* and *HDAC10* encoding plasmid and treated with doxorubicin. The knockdown of *HSC70* expression completely abolished the cell death protective function of HDAC10 (Fig.. 9D), indicating that indeed HDAC10 promotes resistance to doxorubicin via intact CMA. Finally, we investigated the tumor selectivity of HDAC10 mediated cell survival using astrocyte cultures. Because these cells are hard to transfect, we treated cells with the class IIb specific inhibitor bufexamac and compared metabolic activity of NB cells with astrocytes both co-treated with doxorubicin. Whereas co-treatment of bufexamac and low-dose doxorubicin strongly reduced metabolic activity in BE(2)C cells, this was not the case in normal astrocytes (Fig.. 9E). However, the effect of bufexamac in NB cells was rather low, compared to the knockdown of HDAC10 expression in BE(2)C cells, which may be a consequence of unspecificity, because bufexamac has been described as a selective inhibitor for HDAC6 and 10, with a preference for targeting HDAC6 (Bantscheff et al., op. cit.).

In summary, inhibition of autophagic flux via interference with lysosomal functions through targeting of HDAC10 enhanced tumor-specific toxicity of DNA-damage inducing drugs and may serve as a promising approach for co-treatment of therapy resistant tumors.

### Example 6: High HDAC10 expression correlates with poor survival of neuroblastoma patients

The experimental data demonstrate that HDAC10 regulates survival of neuroblastoma cells and mediates resistance to cytotoxic drugs; hence, it was next asked whether HDAC10 expression correlates with clinical outcome of neuroblastoma with the help of the web-based R2 microarray data base. Indeed, high *HDAC10* expression significantly correlated with poor overall survival (Fig.. 10A(a),(b)) with a significant up-regulation in tumor samples of patients not alive (Fig.. 10A(c)). Additionally, high *HDAC10* expression correlated with recurrence or progression (Fig.. 10A(d)). Furthermore, HDAC10 correlation calculations revealed that ATG4D, which is necessary for autophagosome formation during autophagy induction, is among the top correlated genes (Fig.. 10A(e)) and *ATG4D* expression itself was also significantly correlated with poor overall survival (Fig.. 10A(f)). Of note, NB cells endogenously expressed HDAC10 in levels reflecting the *in vivo* situation in the tumor (Fig 10A(g)).

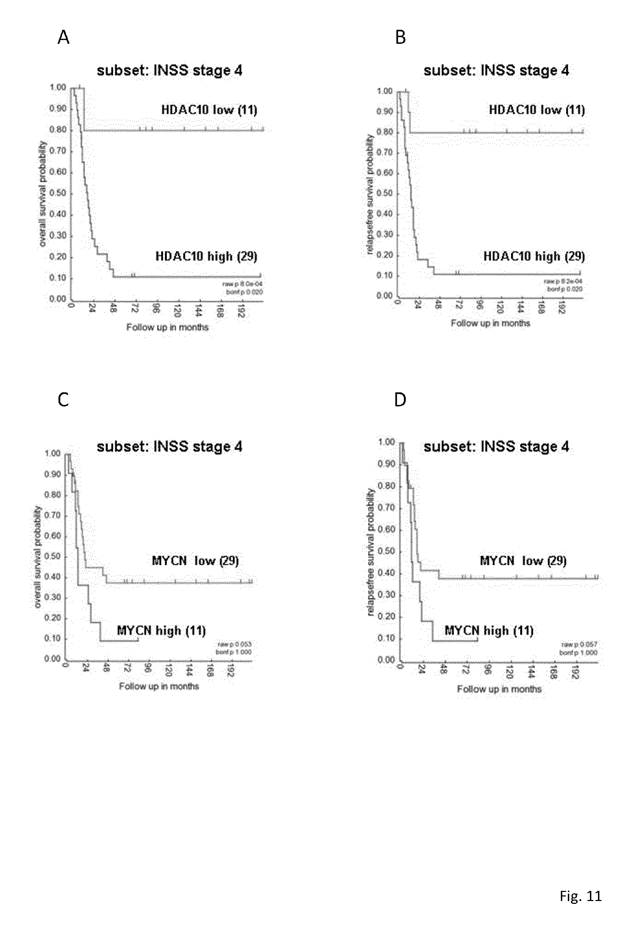

Additionally, high *HDAC10* expression significantly correlated with poor overall (Fig. 11A) as well as relapse free (Fig. 11B) survival in a cohort of high-risk INSS stage 4 patients (treated with multimodal chemotherapy), suggesting that HDAC10-mediated autophagy induction might also function as a survival mechanism against cytotoxic drug treatment in highly resistant neuroblastoma tumors *in vivo*. In comparison, MYCN expression is not suitable marker to significantly discriminate the high-risk group (Fig. 11C,D). Taken together, *HDAC10* expression significantly separates the survival probability of high-risk stage 4 patients and elevated *HDAC10* expression levels correlate with recurrence or progression.

**Table 1: Selectivity of applied HDAC inhibitors**

| | **type of HDAC inhibitor** | **applied concentration** * | **IC50 HDAC10 from RBC** | **IC50 HDAC10 from literature** | **References** |
|---|---|---|---|---|---|
| **VPA** | Class I, IIa, IV | 1.5 mM | > 10 mM ** | | Gruvich et al. |
| **Compound2** | HDAC8 selective | 40 µM | > 50 µM *** | | KrennHrubec et al. |
| **TSA** | Class I, IIa, IIb, IV | 75 nM | 30 nM | | Khan et al. |
| **Vorinostat (SAHA)** | Class I, IIa, IIb, IV | 0.5 µM | 199 nM | | |
| **PCI-24781** | Class I, IIa, IIb, IV | 0.5 µM | 0.24 µM | | Balasubramanian et al. |
| **Panobinostat (LBH589)** | Class I, IIa, IIb, IV | 100 nM | | 2.3 nM | Atadja |
| **Entinostat (MS-275)** | HDAC1, 2, 3, 9 | 250 nM | | 817 nM | Hess-Stumpp et al. |
| | | | | | Takai & Narahara |
| **Tubacin** | HDAC6 selective | 5 µM | | 12 µM | Haggarty et al. |
| **Bufexamac** | Class IIb selective | 100 µM | | 12.3 µM | Bantscheff et al. |

| | | | | | |
|---|---|---|---|---|---|
| * applied concentration in cell culture experiments ** The calculated IC50-value of VPA is above the highest concentration included in the assay (10 mM). Due to the dose-response relationship, we can calculate a theoretical inhibition for HDAC10 0 of less than 0.1 % for the concentration applied in cell culture (1 mM VPA). *** The dose-response relationship for Compound2 is characterized by a poor goodness of fit (R²=0.4651) as well as a large confidence interval. Therefore, no trustable IC50 estimation can be given. However, at the highest applied concentration of 50 µM, residual HDAC10 0 activity was still > 86%. RBC: Reaction Biology Corporation (Malven, PA, USA) | | | | | |

## Claims

1. A method of identifying a subject being susceptible to a co-treatment with an histone deacetylase 10 (HDAC10)-inhibitor, said subject being in need of and/or being treated with cancer therapy against a cancer, comprising the steps of
a) determining the amount of an HDAC10 gene product and/or an autophagy-related gene 4D (ATG4D) product in a cancer sample from said subject,
b) comparing the amount of said HDAC10 gene product as determined in step a) with a reference amount for said HDAC10 gene product and/or the amount of ATG4D product as determined in step a) with a reference amount for said ATG4D product, and
c) identifying a subject being susceptible to co-treatment with an HDAC10-inhibitor.

2. The method of claim 1, wherein the HDAC10-inhibitor is a specific inhibitor of class IIb histone deacetylases.

3. The method of claim 1 or 2, wherein the HDAC10-inhibitor is 2-(4-butoxyphenyl)-N-hydroxyacetamide (bufexamac).

4. The method of any one of claims 1 to 3, wherein an amount of HDAC10 gene product higher than the reference amount for said HDAC10 gene product and/or an amount of ATG4D product higher than the reference amount for said ATG4D product indicates that said subject is susceptible to co-treatment with an HDAC10 inhibitor.

5. A method of predicting the risk of recurrence and/or progression of a cancer in an subject treated by cancer therapy, comprising the steps of
a) determining the amount of an HDAC10 gene product and/or an ATG4D product in a cancer sample from said subject,
b) comparing the amount of said HDAC10 gene product as determined in step a) with a reference amount for said HDAC10 gene product and/or the amount of ATG4D product as determined in step a) with a reference amount for said ATG4D product, and
c) predicting the risk of recurrence and/or progression of a cancer treated by cancer therapy.

6. The method of claim 5, wherein an amount of HDAC10 gene product higher than the reference amount for said HDAC10 gene product and/or an amount of ATG4D product higher than the reference amount for ATG4D product indicates a high risk of recurrence and/or progression of said tumor.

7. The method of any one of claims 1 to 6, wherein the amount of HDAC10 is determined by immunological methods.

8. The method of any one of claims 1 to 6, wherein the amount of HDAC10 is determined by a method comprising
a) extracting RNA from said cancer sample, and
b) hybridizing polynucleic acids derived from said RNA to at least one probe specific for HDAC10 or ATG4D.

9. A device for identifying a subject being susceptible to co-treatment with an HDAC10-inhibitor, comprising an analyzing unit for determining the amount of an HDAC10 gene product and/or ATG4D product in a cancer sample from a subject in need of and/or treated by cancer therapy against a cancer and an evaluation unit for comparing the amount determined by said analyzing unit to a reference amount for said HDAC10 gene product and/or ATG4D product, whereby a subject being susceptible to co-treatment with an HDAC10-inhibitor is identified.

10. A kit adapted to carry out the method of any one of claims 1 to 8, said kit comprising instructions for carrying out the said method, and means for determining the amount of an HDAC10 gene product and/or ATG4D product in a sample from a subject in need of cancer therapy against a neurological tumor, and means for comparing the amount determined by said means to a reference amount for said HDAC10 gene product and/or ATG4D product allowing identifying a subject being susceptible to co-treatment with an HDAC10-inhibitor.

11. 2-(4-butoxyphenyl)-N-hydroxyacetamide (bufexamac) for use in treating cancer in a subject suffering therefrom, wherein a sample from said cancer was determined to comprise a high amount of HDAC10 gene product as compared to a reference amount for said HDAC10 gene product and/or a high amount of ATG4D product as compared to a reference amount for said ATG4D product and wherein said bufexamac is to be applied in a co-treatment with a chemotherapeutic agent.

12. The method of any one of claims 1 to 8, the device of claim 9, the kit of claim 10, or the bufexamac for use in a co-treatment of claim 11, wherein the cancer is a neurological or neuroendocrine tumor.

13. The method, device, kit, or bufexamac for use in a co-treatment of claim 12, wherein the tumor is a neuroblastoma.

14. The method, device, kit, or bufexamac for use in a co-treatment of claim 12, wherein the tumor is a neuroblastoma grade IV.

15. The method of any one of claims 1 to 8, the device of claim 9, the kit of claim 10, or the bufexamac for use in a co-treatment of claim 11, wherein the chemotherapeutic agent is selected from the group consisting of doxorubicin, epirubicin, idarubicin, daunorubicin, irinotecan, topotecan, camptothecin, VP16, platinum, vincristin, cyclophosphamid, ifosfamide, dacarbazine, and vindesine.
